# EUROPEAN PATENT SPECIFICATION

(11) **EP 4 249 507 B1**
(45) Date of publication and mention of the grant of the patent: **12.08.2026**
(21) Application number: 23175859.0
(22) Date of filing: 11.03.2014
(51) Int. Cl.: A01K 67/0278, C07K 14/74

(54) **TRANSGENIC MICE EXPRESSING CHIMERIC MAJOR HISTOCOMPATIBILITY COMPLEX (MHC) CLASS II MOLECULES**
TRANSGENE MÄUSE, DIE CHIMÄRE MHC-KLASSE-II-MOLEKÜLE EXPRIMIEREN
SOURIS TRANSGÉNIQUES EXPRIMANTS DES MOLÉCULES DU COMPLEXE MAJEUR D'HISTOCOMPATIBILITÉ (CMH) DE CLASSE II CHIMÉRIQUES

(30) Priority: 11.03.2013 US 201313793935
(43) Date of publication of application: 27.09.2023
(60) Divisional application: 26189923.1
(62) Document number(s) of the earlier application(s) in accordance with Art. 76 EPC: 14718815.5 / 2 967 015
(73) Proprietor: Regeneron Pharmaceuticals, Inc., Tarrytown, NY 10591 (US)
(72) Inventor: MACDONALD, Lynn, Tarrytown, NY 10591 (US); MURPHY, Andrew, J., Tarrytown, NY 10591 (US); TU, Naxin, Tarrytown, NY 10591 (US); GURER, Cagan, Chappaqua, NY 10514 (US); VORONINA, Vera, North Bethesda, MD 20852 (US); STEVENS, Sean, Del Mar, CA 92014 (US)
(74) Representative: Mewburn Ellis LLP

(56) References cited:
- WO-A1-2013/063340
- WO-A1-2014/130667
- ITO K ET AL: "HLA-DR4-IE CHIMERIC CLASS II TRANSGENIC, MURINE CLASS II-DEFICIENT MICE ARE SUSCEPTIBLE TO EXPERIMENTAL ALLERGIC ENCEPHALOMYELITIS", THE JOURNAL OF EXPERIMENTAL MEDICINE, ROCKEFELLER UNIVERSITY PRESS, US, vol. 183, no. 6, 1 June 1996 (1996-06-01), pages 2635 - 2644, XP000994711, ISSN: 0022-1007, DOI: 10.1084/JEM.183.6.2635
- GRETE SØNDERSTRUP ET AL: "HLA class II transgenic mice: models of the human CD4+ T-cell immune response", IMMUNOLOGICAL REVIEWS, WILEY-BLACKWELL PUBLISHING, INC, US, vol. 172, no. 1, 28 April 2006 (2006-04-28), pages 335 - 343, XP071454750, ISSN: 0105-2896, DOI: 10.1111/J.1600-065X.1999.TB01377.X
- A. WOODS ET AL: "Human major histocompatibility complex class II-restricted T cell responses in transgenic mice", JOURNAL OF EXPERIMENTAL MEDICINE, vol. 180, no. 1, 1 July 1994 (1994-07-01), pages 173 - 181, XP055127351, ISSN: 0022-1007, DOI: 10.1084/jem.180.1.173

## Description

### FIELD OF THE INVENTION

Present invention relates to a mouse that is genetically engineered to express a humanized Major Histocompatibility Complex (MHC) class II protein, as well as embryos, tissues, and cells expressing the same. The invention further relates to methods for making a genetically modified mouse that expresses a humanized MHC II protein. Also disclosed are methods for using mice, cells, and tissues that express a humanized MHC class II protein for identifying peptides that activate lymphocytes and engage T cells, and for developing human vaccines and other therapeutics.

### BACKGROUND OF THE INVENTION

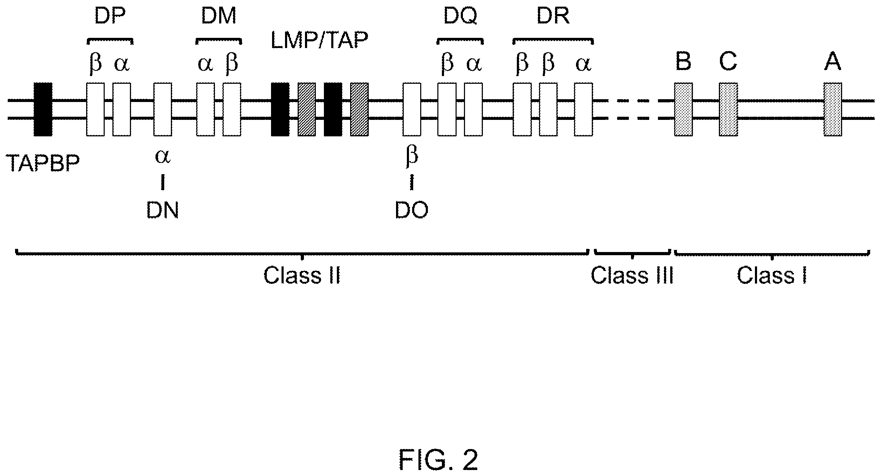

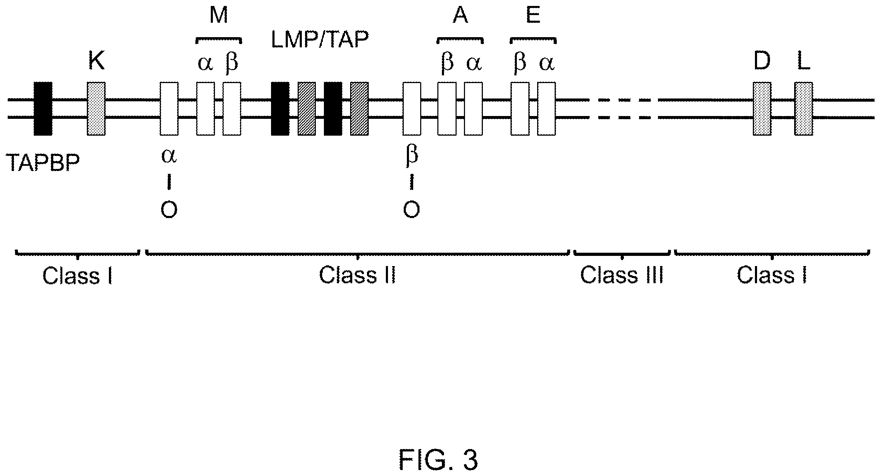

In the adaptive immune response, foreign antigens are recognized by receptor molecules on B lymphocytes (e.g., immunoglobulins) and T lymphocytes (e.g., T cell receptor or TCR). These foreign antigens are presented on the surface of cells as peptide fragments by specialized proteins, generically referred to as major histocompatibility complex (MHC) molecules. MHC molecules are encoded by multiple loci that are found as a linked cluster of genes that spans about 4 Mb. In mice, the MHC genes are found on chromosome 17, and for historical reasons are referred to as the histocompatibility 2 (H-2) genes. In humans, the genes are found on chromosome 6 and are called human leukocyte antigen (HLA) genes. The loci in mice and humans are polygenic; they include three highly polymorphic classes of MHC genes (class I, II and III) that exhibit similar organization in human and murine genomes (see FIG. 2 and FIG. 3, respectively).

MHC loci exhibit the highest polymorphism in the genome; some genes are represented by >300 alleles (e.g., human HLA-DRβ and human HLA-B). All class I and II MHC genes can present peptide fragments, but each gene expresses a protein with different binding characteristics, reflecting polymorphisms and allelic variants. Any given individual has a unique range of peptide fragments that can be presented on the cell surface to B and T cells in the course of an immune response.

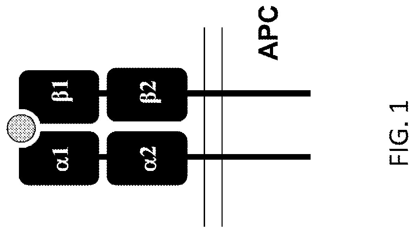

Both humans and mice have class II MHC genes (see FIGs. 2 and 3). In humans, the classical MHC II genes are termed HLA-DP, HLA-DQ, and HLA-DR, whereas in mice they are H-2A and H-2E (often abbreviated as I-A and I-E, respectively). Additional proteins encoded by genes in the MHC II locus, HLA-DM and HLA-DO in humans, and H-2M and H-2O in mice, are not found on the cell surface, but reside in the endocytic compartment and ensure proper loading of MHC II molecules with peptides. Class II molecules consist of two polypeptide chains: α chain and β chain. The extracellular portion of the α chain contains two extracellular domains, α1 and α2; and the extracellular portion of the β chain also contains two extracellular domains, β1 and β2 (see FIG. 1). The α and the β chains are non-covalently associated with each other.

MHC class II molecules are expressed on antigen-presenting cells (APCs), e.g., B cells, macrophages, dendritic cells, endothelial cells during a course of inflammation, etc. MHC II molecules expressed on the surface of APCs typically present antigens generated in intracellular vesicles to CD4+ T cells. In order to participate in CD4+ T cell engagement, the MHC class II complex with the antigen of interest must be sufficiently stable to survive long enough to engage a CD4+ T cell. When a CD4+ T helper cell is engaged by a foreign peptide/MHC II complex on the surface of APC, the T cell is activated to release cytokines that assist in immune response to the invader.

Not all antigens will provoke T cell activation due to tolerance mechanisms. However, in some diseases (e.g., cancer, autoimmune diseases) peptides derived from self-proteins become the target of the cellular component of the immune system, which results in destruction of cells presenting such peptides. There has been significant advancement in recognizing antigens that are clinically significant (e.g., antigens associated with various types of cancer). However, in order to improve identification and selection of peptides that will provoke a suitable response in a human T cell, in particular for peptides of clinically significant antigens, there remains a need for *in vivo* and *in vitro* systems that mimic aspects of human immune system. Thus, there is a need for biological systems (e.g., genetically modified non-human animals and cells) that can display components of a human immune system.

### SUMMARY OF THE INVENTION

A biological system for generating or identifying peptides that associate with human MHC class II proteins and chimeras thereof, and bind to CD4+ T cells, is provided. Mice comprising mouse cells that express humanized molecules that function in the cellular immune response are provided. Humanized mouse loci that encode humanized MHC II proteins are also disclosed. Humanized rodent cells that express humanized MHC molecules are also provided. *In vivo* and *in vitro* systems are provided that comprise humanized rodent cells, wherein the rodent cells express one or more humanized immune system molecules. The invention is as defined in the claims.

The invention relates to a mouse comprising in its genome a nucleotide sequence encoding a humanized MHC II complex, wherein a human portion of the humanized MHC II complex comprises an extracellular domain of a human MHC II complex, e.g., a humanized MHC II α extracellular domain and a humanized MHC II β extracellular domain.

Disclosed herein is a mouse comprising at an endogenous MHC II α gene locus a nucleotide sequence encoding a chimeric human/mouse MHC II α polypeptide. In one example, a human portion of such a chimeric human/mouse MHC II α polypeptide comprises a human MHC II α extracellular domain. In one example, the mouse expresses a functional MHC II complex on a surface of a cell of the mouse. In one example, the human MHC II α extracellular domain in the mouse comprises human MHC II α1 and α2 domains; in one example, a mouse portion of the chimeric human/mouse MHC II α polypeptide comprises transmembrane and cytoplasmic domains of an endogenous mouse MHC II α polypeptide. In one embodiment, the nucleotide sequence encoding a chimeric human/mouse MHC II α polypeptide is operably linked to (e.g., is expressed under regulatory control of) endogenous mouse MHC II α promoter and regulatory elements. The human portion of the chimeric polypeptide is derived from HLA-DR2 protein. The non-human animal is a mouse. The mouse comprises at an endogenous MHC II α gene locus a nucleotide sequence encoding a chimeric human/mouse MHC II α polypeptide and further comprises at an endogenous MHC II β gene locus a nucleotide sequence encoding a chimeric human/mouse MHC II β polypeptide. Also disclosed herein is a method of making a genetically modified mouse comprising at an endogenous MHC II α gene locus a nucleotide sequence encoding a chimeric human/mouse MHC II α polypeptide. Such method may comprise replacing at an endogenous MHC II α gene locus a nucleotide sequence encoding an endogenous mouse MHC II α polypeptide with a nucleotide sequence encoding a chimeric human/mouse MHC II α polypeptide.

Also disclosed herein is a mouse comprising at an endogenous MHC II β gene locus a nucleotide sequence encoding a chimeric human/mouse MHC II β polypeptide. In one example, a human portion of such chimeric human/mouse MHC II β polypeptide comprises a human MHC II β extracellular domain. In one example, the mouse expresses a functional MHC II complex on a surface of a cell of the mouse. In one embodiment, the human MHC II β extracellular domain in the mouse comprises human MHC II β1 and β2 domains; in one example, a non-human portion of the chimeric human/mouse MHC II β polypeptide comprises transmembrane and cytoplasmic domains of an endogenous mouse MHC II β polypeptide. In one embodiment, the nucleotide sequence encoding a chimeric human/mouse MHC II β polypeptide is operably linked to (e.g., is expressed under regulatory control of) endogenous mouse MHC II β promoter and regulatory elements. The human portion of the chimeric polypeptide is derived from HLA-DR2 protein. The non-human animal is a mouse. In one example, the mouse comprising at an endogenous MHC II β gene locus a nucleotide sequence encoding a chimeric human/mouse MHC II β polypeptide further comprises at an endogenous MHC II α gene locus a nucleotide sequence encoding a chimeric human/mouse MHC II α polypeptide. Also provided herein is a method of making a genetically modified mouse comprising at an endogenous MHC II β gene locus a nucleotide sequence encoding a chimeric human/mouse MHC II β polypeptide. Such method may comprise replacing at an endogenous MHC II β gene locus a nucleotide sequence encoding an endogenous mouse MHC II β polypeptide with a nucleotide sequence encoding a chimeric human/mouse MHC II β polypeptide.

Disclosed herein is a mouse comprising at an endogenous MHC II gene locus a first nucleotide sequence encoding a chimeric human/mouse MHC II α polypeptide and a second nucleotide sequence encoding a chimeric human/mouse MHC II β polypeptide, wherein a human portion of the chimeric human/mouse MHC II α polypeptide comprises a human MHC II α extracellular domain and a human portion of the chimeric human/mouse MHC II β polypeptide comprises a human MHC II β extracellular domain. In one example, the chimeric human/mouse MHC II α and β polypeptides form a functional chimeric MHC II complex (e.g., human/mouse MHC II complex) on a surface of a cell. In one example, the human MHC II α extracellular domain comprises human α1 and α2 domains of human MHC II. In one example, the human MHC II β extracellular domain comprises human β1 and β2 domains of human MHC II. In various examples, the first nucleotide sequence is expressed under regulatory control of (e.g., is operably linked to) endogenous non-human MHC II α promoter and regulatory elements. In various examples, the second nucleotide sequence is expressed under regulatory control of (e.g., is operably linked to) endogenous mouse MHC II β promoter and regulatory elements. In some examples, a mouse portion of the chimeric human/mouse MHC II α polypeptide comprises transmembrane and cytoplasmic domains of an endogenous mouse MHC II α polypeptide. In some examples, a mouse portion of the chimeric human/mouse MHC II β polypeptide comprises transmembrane and cytoplasmic domains of an endogenous mouse MHC II β polypeptide.

The non-human animal is a mouse, and the human portions of the chimeric human/mouse MHC II α and β polypeptides comprise human sequences derived from a human HLA-DR2 sequence

In various examples, the first and the second nucleotide sequences are located on the same chromosome. In some examples, the mouse comprises two copies of the MHC II locus containing the first and the second nucleotide sequences, while in other aspects, the mouse comprises one copy of the MHC II locus containing the first and the second nucleotide sequences. Thus, the mouse may be homozygous or heterozygous for the MHC II locus containing the first and the second nucleotide sequences. In one example, the chimeric MHC II described herein is in the germline of a mouse.

In some examples, the chimeric MHC II α polypeptide and/or the chimeric MHC II β polypeptide is operably linked to a mouse leader sequence.

The genetically engineered non-human animal is a mouse. Thus, non-human sequences of the chimeric MHC II α and β genes are derived from nucleotide sequences encoding mouse MHC II protein, e.g., mouse H-2A or H-2E protein. In one embodiment, the mouse of the invention does not express functional endogenous MHC II polypeptides from its endogenous loci. In one embodiment, the mouse does not express functional endogenous H-2E and H-2A polypeptides from its endogenous loci.

Thus, disclosed herein is a mouse comprising at an endogenous mouse MHC II locus a first nucleotide sequence encoding a chimeric human/mouse MHC II α polypeptide and a second nucleotide sequence encoding a chimeric human/mouse MHC II β polypeptide, wherein a human portion of the chimeric MHC II α polypeptide comprises an extracellular domain derived from an α polypeptide of HLA-DR2 and a human portion of the chimeric human/mouse MHC II β polypeptide comprises an extracellular domain derived from a β polypeptide of HLA-DR2, wherein a mouse portion of the chimeric MHC II α polypeptide comprises transmembrane and cytoplasmic domains of a mouse H-2A or H-2E α chain and a mouse portion of the chimeric MHC II β polypeptide comprises transmembrane and cytoplasmic domains of a mouse H-2A or H-2E β chain, and wherein the mouse expresses a functional chimeric HLA class II complex. In another embodiment, the functional chimeric HLA class II complex is HLA-DR2/H-2E. The extracellular domain of the chimeric MHC II α polypeptide comprises human α1 and α2 domainsand the extracellular domain of the chimeric MHC II β polypeptide comprises human β1 and β2 domains. In some embodiments, the first nucleotide sequence is expressed under regulatory control of (e.g., is operably linked to) endogenous mouse MHC II α promoter and regulatory elements, and the second nucleotide sequence is expressed under regulatory control of (e.g., is operably linked to) endogenous mouse MHC II β promoter and regulatory elements. In various embodiments, the mouse does not express functional endogenous MHC II polypeptides, e.g., H-2E and H-2A polypeptides, from their endogenous loci. In some aspects, the mouse comprises two copies of the MHC II locus containing the first and the second nucleotide sequences, while in other aspects, the mouse comprises one copy of the MHC II locus containing the first and the second nucleotide sequences.

Additionally, provided herein is a mouse that comprises in its genome, e.g., at its endogenous MHC II locus, one or more, e.g., one, two, three, four, nucleotide sequence(s) encoding a chimeric human/mouse MHC II complex(es). In one embodiment, each complex comprises an MHC II α and an MHC II β polypeptides, and a human portion of the chimeric MHC II α and MHC II β polypeptides comprises an extracellular domain of a human MHC II α and MHC II β polypeptides, respectively, while a mouse portion comprises transmembrane and cytoplasmic domains of a mouse MHC II α and MHC II β polypeptides, respectively. Thus, the mouse may express one or more, e.g., one, two, three, four, chimeric human/mouse MHC II complex(es).

Methods of making genetically engineered mice as described herein are also disclosed. In various examples, mice of the invention are made by replacing endogenous MHC II sequences with nucleotide sequences encoding chimeric human/mouse MHC II α and β polypeptides. In one example, the invention provides a method of modifying an MHC II locus of a mouse to express a chimeric human/mouse MHC II complex comprising replacing at the endogenous mouse MHC II locus a nucleotide sequence encoding a mouse MHC II complex with a nucleotide sequence encoding a chimeric human/mouse MHC II complex. In one example of the method, the nucleotide sequence encoding the chimeric human/mouse MHC II complex comprises a first nucleotide sequence encoding an extracellular domain of a human MHC II α chain and transmembrane and cytoplasmic domains of a mouse MHC II α chain and a second nucleotide sequence encoding an extracellular domain of a human MHC II β chain and transmembrane and cytoplasmic domains of a mouse MHC II β chain. In some aspects, a mouse portion of the chimeric MHC II complex is derived from a mouse H-2E protein, and a human portion is derived from a human HLA-DR2 protein. In some examples, the replacement of the endogenous MHC II loci described herein is made in a single ES cell, and the single ES cell is introduced into a mouse embryo to make a genetically modified mouse.

Also disclosed herein is a mouse chimeric MHC II locus encoding a chimeric human/mouse MHC II complex, comprising a first nucleotide sequence encoding an extracellular domain of a human MHC II α chain and transmembrane and cytoplasmic domains of a mouse MHC II α chain and a second nucleotide sequence encoding an extracellular domain of a human MHC II β chain and transmembrane and cytoplasmic domains of a mouse MHC II β chain. In one example, the chimeric MHC II locus is at an endogenous MHC II position in a genome of a mouse. In one aspect, the chimeric MHC II locus expresses a chimeric human/mouse MHC II complex. The human MHC II is HLA-DR2. The mouse MHC II is selected from H-2A and H-2E. The chimeric MHC II locus may be obtainable by any methods described herein for generating genetically modified mice.

Also disclosed herein are cells, e.g., isolated antigen-presenting cells, derived from the mice described herein. Tissues and embryos derived from the mice described herein are also disclosed.

Any of the embodiments and aspects described herein can be used in conjunction with one another, unless otherwise indicated or apparent from the context.

### BRIEF DESCRIPTION OF THE DRAWINGS

**FIG. 1** is a schematic drawing of the MHC II class molecule expressed on the surface of an antigen presenting cell (APC), containing four domains: α1, α2, β1, and β2. The gray circle represents a peptide bound in the peptide-binding cleft.
**FIG. 2** is a schematic representation (not to scale) of the relative genomic structure of the human HLA, showing class I, II and III genes.
**FIG. 3** is a schematic representation (not to scale) of the relative genomic structure of the mouse MHC, showing class I, II and III genes.

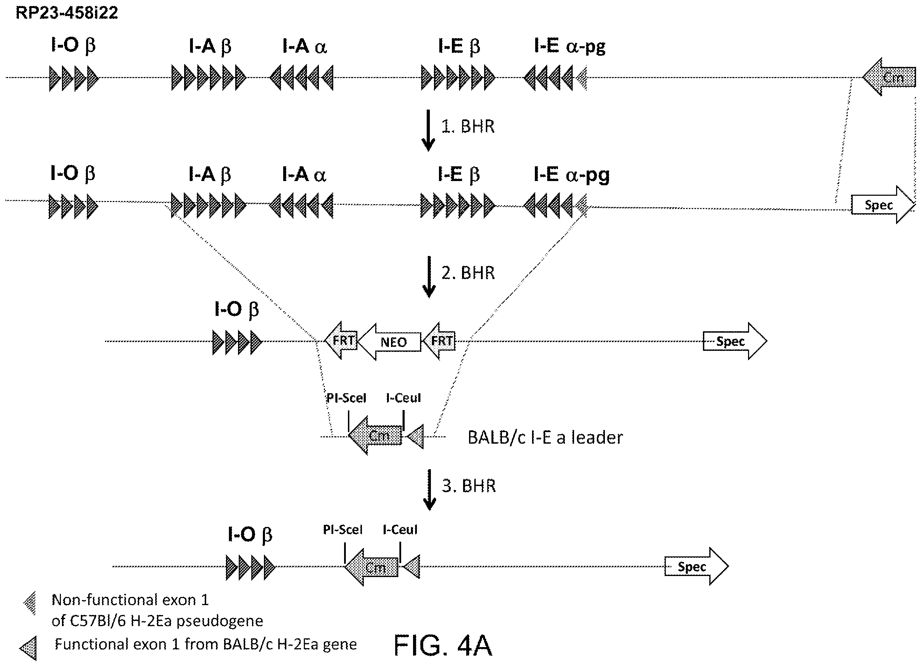

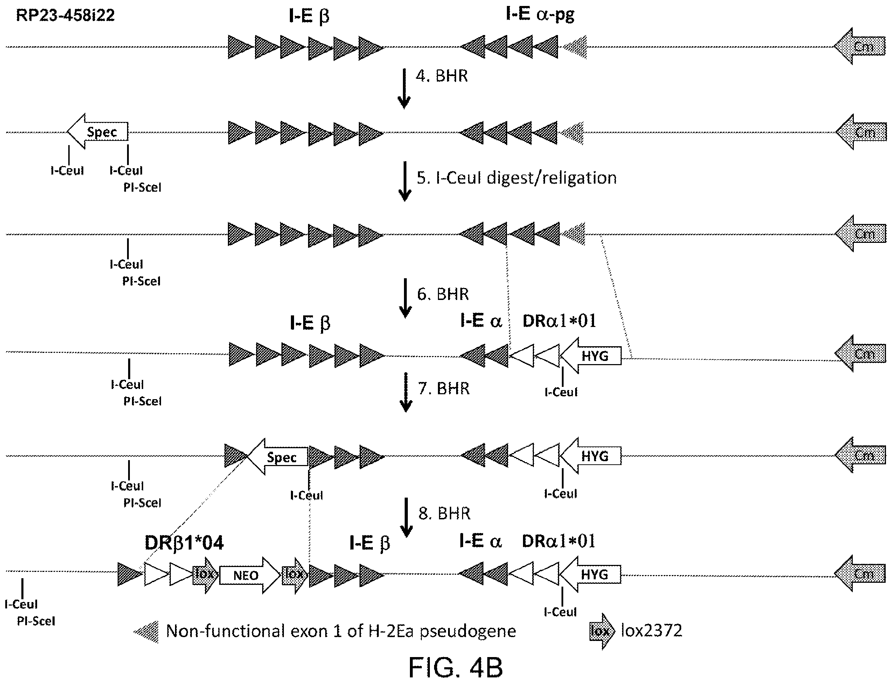

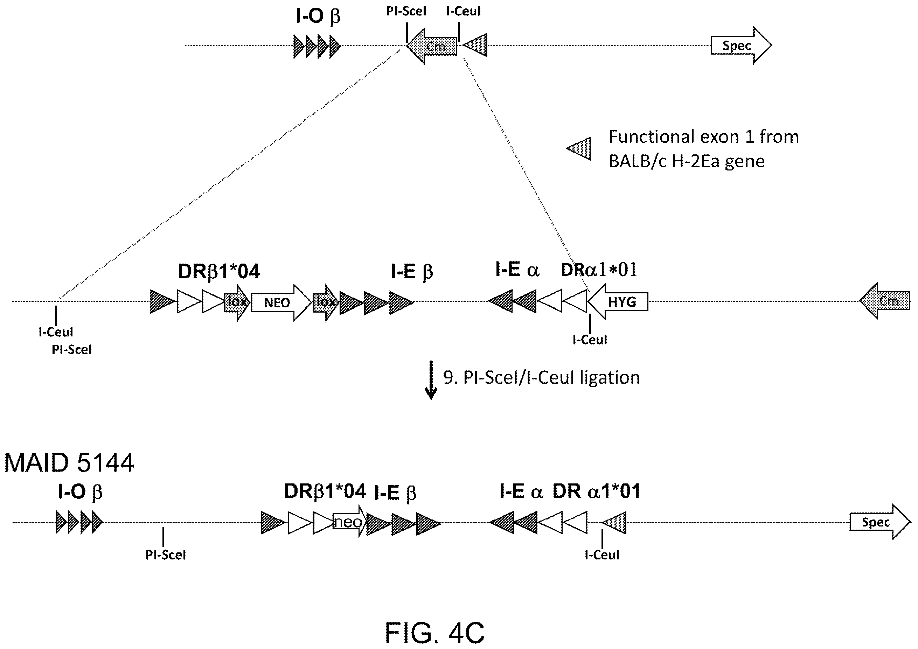

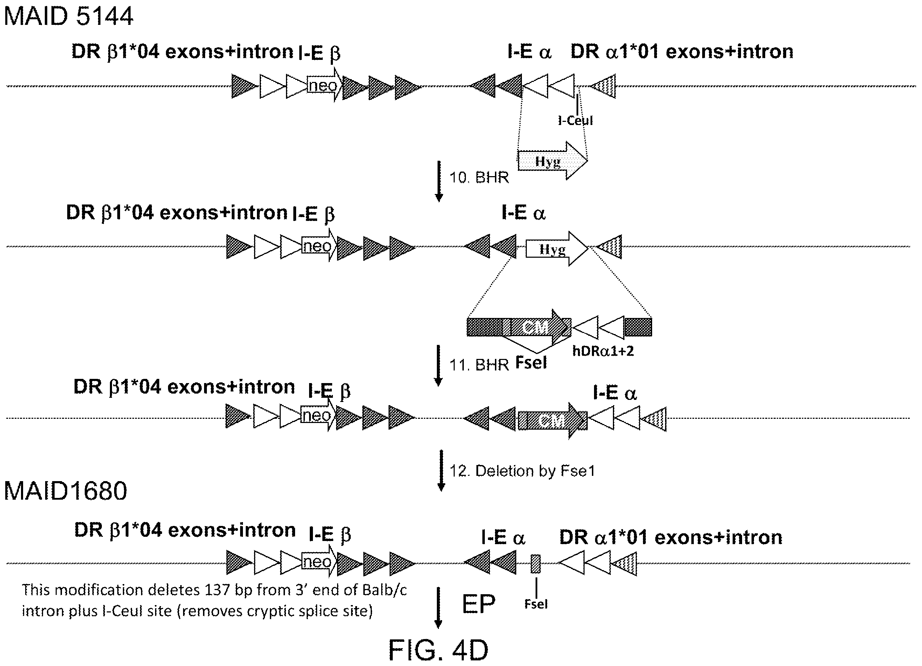

**FIG. 4** **(A-D)** is a schematic illustration (not to scale) of the strategy for generating a targeting vector comprising humanized I-E β and I-E α (i.e., H-2Eβ/HLA-DRβ1*04 and H-2Eα/HLA-DRα*01 chimera, respectively). In FIG. 4C, the final humanized MHC II sequence from FIG. 4B is ligated between PI-SceI and I-CeuI restriction sites of the final construct from FIG. 4A, to generate a construct comprising humanized MHC II and exon 1 of I-Eα from BALB/c. Pg=pseudogene; BHR= bacterial homologous recombination; CM=chloramphenicol; spec=spectinomycin; hyg=hygromycin; neo=neomycin; EP=electroporation. Triangles represent exons, filled triangles represent mouse exons from C57BL/6 mouse (with the exception of hashed triangles, which represent exon 1 of I-Eα from BALB/c mouse) and open triangles represent human exons.
**FIG. 5** shows a schematic illustration, not to scale, of MHC class II I-E and I-A genes, showing knockout of the mouse locus using a hygromycin cassette, followed by introduction of a vector comprising a humanized I-E β and I-E α (i.e., H-2Eβ/HLA-DRβ1*04 and H-2Eα/HLA-DRα*01 chimera, respectively). Open triangles represent human exons; filled triangles represent mouse exons. Probes used for genotyping are encircled.
**FIG. 6** shows a schematic illustration, not to scale, of Cre-mediated removal of the neomycin cassette of FIG. 5. Open triangles represent human exons; filled triangles represent mouse exons. Top two strands represent MHC II loci in humanized MHC II heterozygous mouse harboring a neomycin selection cassette, and bottom two strands represent MHC II loci in humanized MHC II heterozygous mouse with neomycin cassette removed.

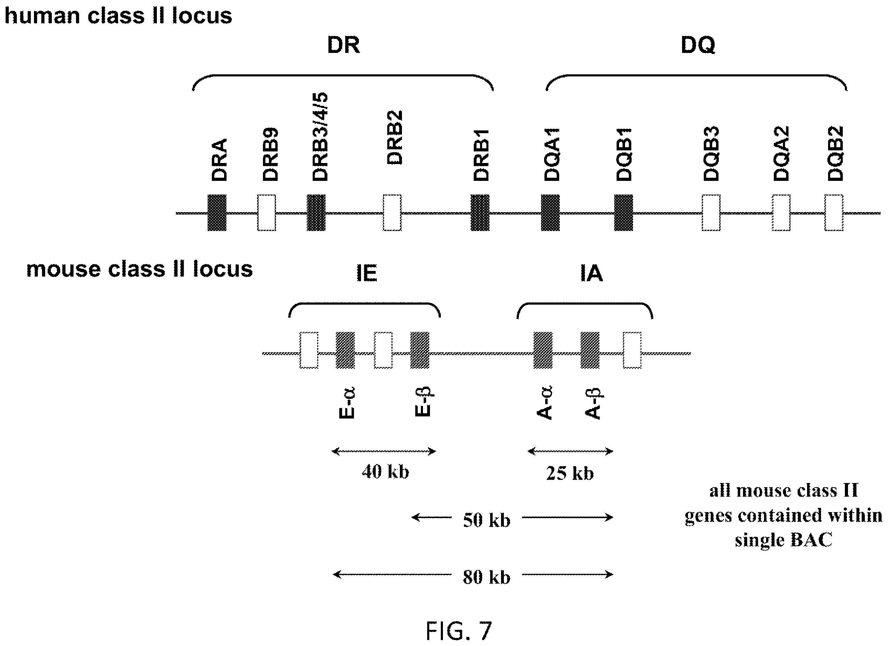

**FIG. 7** shows a schematic comparative illustration, not to scale, of mouse and human class II loci. Class II genes are represented by boxes. Relative sizes (kb) of various nucleic acid fragments are included.

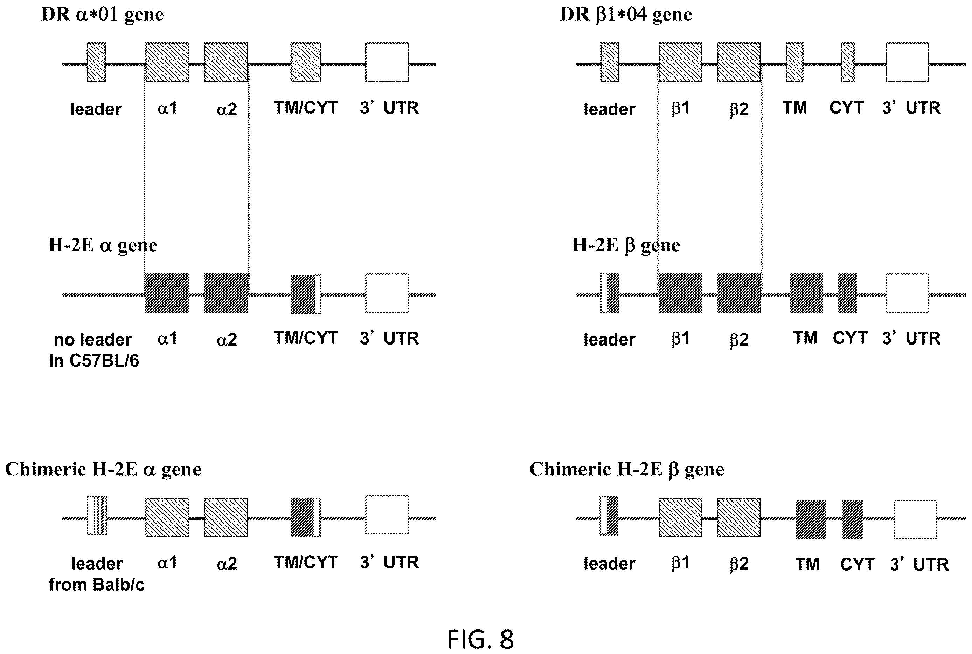

**FIG. 8****,** at left panel, is a schematic illustration (not to scale) of humanization strategy for the MHC II α chain; in particular, the figure shows a replacement of α1 and α2 domains, encoded by exons 2 and 3 of MHC II α gene, while retaining mouse transmembrane and cytoplasmic tail sequences. In the humanized locus, the MHC II α leader sequence is derived from the mouse BALB/c strain. The right panel illustrates humanization of the MHC II β chain; in particular, the figure shows a replacement of β1 and β2 domains, encoded by exons 2 and 3 of MHC II β gene, while retaining the mouse leader and mouse transmembrane and cytoplasmic tail sequences. Top row are all human sequences; middle row are all mouse sequences; bottom row are all humanized sequences, with exons 2 and 3 derived from human HLA-DR genes.
**FIG. 9** shows FACS analysis with anti-HLA-DR antibody of B cells from a mouse heterozygous for a chimeric HLA-DR4 (neo cassette removed) in the presence (1681HET + poly(I:C) or absence (1681HET) of poly(I:C), and a wild-type mouse (WT mouse).

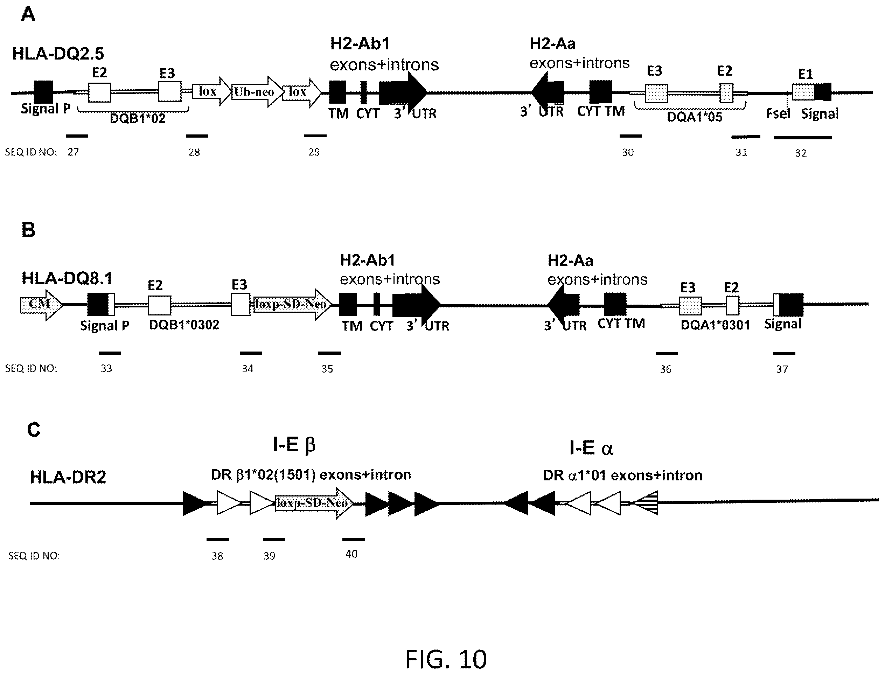

**FIG. 10** is a schematic illustration (not to scale) of the targeting vectors used for generating humanized MHC II genes, specifically, a targeting vector for generating HLA-DQ2.5/H2-A mouse **(****FIG. 10A****),** a targeting vector for generating HLA-DQ8.1/H-2A mouse **(****FIG. 10B****),** and a targeting vector for generating HLA-DR2/H-2E mouse **(****FIG. 10C****).** In the diagrams, unless indicated otherwise (e.g., *loxP* site, etc.), the empty boxes or triangles are sequences of the human exons, the double lines are sequences of the human introns, the filed boxes or triangles are sequences of the mouse exons, single lines are sequences of mouse introns, and the hashed triangle is exon 1 of I-Eα from BALB/c mouse. Location of junctional sequences are indicated below each targeting vector diagram and presented in Table 1 and the Sequence Listing.
**FIG. 11** shows mouse H-2A/H-2E (IA/IE) and human HLA-DQ2.5 expression on CD19+ B cells obtained from WT or heterozygous HLA-DQ2.5/H-2A ("6040Het") mice.

### DETAILED DESCRIPTION OF THE INVENTION

### Definitions

The present invention relates to genetically modified mice that express human or humanized MHC II polypeptide; cells comprising the same; methods of making the same; as well as methods of using the same. Unless defined otherwise, all terms and phrases used herein include the meanings that the terms and phrases have attained in the art, unless the contrary is clearly indicated or clearly apparent from the context in which the term or phrase is used.

The term "conservative," when used to describe a conservative amino acid substitution, includes substitution of an amino acid residue by another amino acid residue having a side chain R group with similar chemical properties (e.g., charge or hydrophobicity). Conservative amino acid substitutions may be achieved by modifying a nucleotide sequence so as to introduce a nucleotide change that will encode the conservative substitution. In general, a conservative amino acid substitution will not substantially change the functional properties of interest of a protein, for example, the ability of MHC II to present a peptide of interest. Examples of groups of amino acids that have side chains with similar chemical properties include aliphatic side chains such as glycine, alanine, valine, leucine, and isoleucine; aliphatic-hydroxyl side chains such as serine and threonine; amide-containing side chains such as asparagine and glutamine; aromatic side chains such as phenylalanine, tyrosine, and tryptophan; basic side chains such as lysine, arginine, and histidine; acidic side chains such as aspartic acid and glutamic acid; and, sulfur-containing side chains such as cysteine and methionine. Conservative amino acids substitution groups include, for example, valine/leucine/isoleucine, phenylalanine/tyrosine, lysine/arginine, alanine/valine, glutamate/aspartate, and asparagine/glutamine. In some embodiments, a conservative amino acid substitution can be a substitution of any native residue in a protein with alanine, as used in, for example, alanine scanning mutagenesis. In some embodiments, a conservative substitution is made that has a positive value in the PAM250 log-likelihood matrix disclosed in Gonnet et al. ((1992) Exhaustive Matching of the Entire Protein Sequence Database, Science 256:1443-45), hereby incorporated by reference. In some embodiments, the substitution is a moderately conservative substitution wherein the substitution has a nonnegative value in the PAM250 log-likelihood matrix.

Thus, also disclosed is a genetically modified mouse whose genome comprises a nucleotide sequence encoding a human or humanized MHC II polypeptide, wherein the polypeptide comprises conservative amino acid substitutions in the amino acid sequence described herein.

One skilled in the art would understand that in addition to the nucleic acid residues encoding a human or humanized MHC II polypeptide described herein, due to the degeneracy of the genetic code, other nucleic acids may encode the polypeptide of the invention. Therefore, in addition to a genetically modified mouse that comprises in its genome a nucleotide sequence encoding MHC II polypeptide with conservative amino acid substitutions, a non-human animal whose genome comprises a nucleotide sequence that differs from that described herein due to the degeneracy of the genetic code is also disclosed.

The term "identity" when used in connection with sequence includes identity as determined by a number of different algorithms known in the art that can be used to measure nucleotide and/or amino acid sequence identity. In some examples described herein, identities are determined using a ClustalW v. 1.83 (slow) alignment employing an open gap penalty of 10.0, an extend gap penalty of 0.1, and using a Gonnet similarity matrix (MacVector^{™} 10.0.2, MacVector Inc., 2008). The length of the sequences compared with respect to identity of sequences will depend upon the particular sequences. In various embodiments, identity is determined by comparing the sequence of a mature protein from its N-terminal to its C-terminal. In various examples when comparing a chimeric human/non-human sequence to a human sequence, the human portion of the chimeric human/non-human sequence (but not the non-human portion) is used in making a comparison for the purpose of ascertaining a level of identity between a human sequence and a human portion of a chimeric human/non-human sequence (e.g., comparing a human ectodomain of a chimeric human/mouse protein to a human ectodomain of a human protein).

The terms "homology" or "homologous" in reference to sequences, e.g., nucleotide or amino acid sequences, means two sequences which, upon optimal alignment and comparison, are identical in at least about 75% of nucleotides or amino acids, e.g., at least about 80% of nucleotides or amino acids, e.g., at least about 90-95% nucleotides or amino acids, e.g., greater than 97% nucleotides or amino acids. One skilled in the art would understand that, for optimal gene targeting, the targeting construct should contain arms homologous to endogenous DNA sequences (i.e., "homology arms"); thus, homologous recombination can occur between the targeting construct and the targeted endogenous sequence.

The term "operably linked" refers to a juxtaposition wherein the components so described are in a relationship permitting them to function in their intended manner. As such, a nucleic acid sequence encoding a protein may be operably linked to regulatory sequences (e.g., promoter, enhancer, silencer sequence, etc.) so as to retain proper transcriptional regulation. In addition, various portions of the chimeric or humanized protein of the invention may be operably linked to retain proper folding, processing, targeting, expression, and other functional properties of the protein in the cell. Unless stated otherwise, various domains of the chimeric or humanized protein of the invention are operably linked to each other.

The terms "MHC II complex," "MHC II protein," or the like, as used herein, include the complex between an MHC II α polypeptide and an MHC II β polypeptide. The term "MHC II α polypeptide" or "MHC II β polypeptide" (or the like), as used herein, includes the MHC II α polypeptide alone or MHC II β polypeptide alone, respectively. Similarly, the terms "HLA-DR4 complex", "HLA-DR4 protein," "H-2E complex," "H-2E" protein," or the like (e.g., similar terms referring to other MHC II alleles), refer to complex between α and β polypeptides. Typically, the terms "human MHC" and "HLA" are used interchangeably.

The term "replacement" in reference to gene replacement refers to placing exogenous genetic material at an endogenous genetic locus, thereby replacing all or a portion of the endogenous gene with an orthologous or homologous nucleic acid sequence. As demonstrated in the Examples below, nucleic acid sequence of endogenous MHC II locus was replaced by a nucleotide sequence comprising sequences encoding portions of human MHC II α and β polypeptides; specifically, encoding the extracellular portions of the MHC II α and β polypeptides.

"Functional" as used herein, e.g., in reference to a functional polypeptide, refers to a polypeptide that retains at least one biological activity normally associated with the native protein. For example, in some embodiments of the invention, a replacement at an endogenous locus (e.g., replacement at an endogenous non-human MHC II locus) results in a locus that fails to express a functional endogenous polypeptide. Likewise, the term "functional" as used herein in reference to functional extracellular domain of a protein, refers to an extracellular domain that retains its functionality, e.g., in the case of MHC II, ability to bind an antigen, ability to bind a T cell co-receptor, etc. In some embodiments of the invention, a replacement at the endogenous MHC locus results in a locus that fails to express an extracellular domain (e.g., a functional extracellular domain) of an endogenous MHC while expressing an extracellular domain (e.g., a functional extracellular domain) of a human MHC.

### Genetically Modified MHC II Animals

Disclosed herein are genetically modified mice that comprise in their genome a nucleotide sequence encoding a human or humanized MHC II complex; thus, the mice express a human or humanized MHC II complex (e.g., MHC II α and β polypeptides).

MHC genes are categorized into three classes: class I, class II, and class III, all of which are encoded either on human chromosome 6 or mouse chromosome 17. A schematic of the relative organization of the human and mouse MHC classes is presented in FIGs. 2 and 3, respectively. The majority of MHC genes are polymorphic, in fact they are the most polymorphic genes of the mouse and human genomes. MHC polymorphisms are presumed to be important in providing evolutionary advantage; changes in sequence can result in differences in peptide binding that allow for better antigen presentation. One exception is the human HLA-DRα chain and its mouse homolog, Eα (i.e., H-2Ea), which are monomorphic.

MHC class II complex comprises two non-covalently associated domains: an α chain and a β chain, also referred herein as an α polypeptide and a β polypeptide (FIG. 1). The protein spans the plasma membrane; thus it contains an extracellular domain, a transmembrane domain, and a cytoplasmic domain. The extracellular portion of the α chain includes α1 and α2 domains, and the extracellular portion of the β chain includes β1 and β2 domains. The α1 and β1 domains form a peptide-binding cleft on the cell surface. Due to the three-dimensional confirmation of the peptide-binding cleft of the MHC II complex, there is theoretically no upper limit on the length of the bound antigen, but typically peptides presented by MHC II are between 13 and 17 amino acids in length.

In addition to its interaction with the antigenic peptides, the peptide-binding cleft of the MHC II molecule interacts with invariant chain (Ii) during the processes of MHC II complex formation and peptide acquisition. The α/β MHC II dimers assemble in the endoplasmic reticulum and associate with Ii chain, which is responsible for control of peptide binding and targeting of the MHC II into endocytic pathway. In the endosome, Ii undergoes proteolysis, and a small fragment of Ii, Class II-associated invariant chain peptide (CLIP), remains at the peptide-binding cleft. In the endosome, under control of HLA-DM (in humans), CLIP is exchanged for antigenic peptides.

MHC II interacts with T cell co-receptor CD4 at the hydrophobic crevice at the junction between α2 and β2 domains. Wang and Reinherz (2002) Structural Basis of T Cell Recognition of Peptides Bound to MHC Molecules, Molecular Immunology, 38:1039-49. When CD4 and T cell receptor bind the same MHC II molecule complexed with a peptide, the sensitivity of a T cell to antigen is increased, and it requires 100-fold less antigen for activation. *See,* Janeway's Immunobiology, 7th Ed., Murphy et al. eds., Garland Science, 2008.

Numerous functions have been proposed for transmembrane and cytoplasmic domains of MHC II. In the case of cytoplasmic domain, it has been shown to be important for intracellular signaling, trafficking to the plasma membrane, and ultimately, antigen presentation. For example, it was shown that T cell hybridomas respond poorly to antigen-presenting cells (APCs) transfected with MHC II β chains truncated at the cytoplasmic domain, and induction of B cell differentiation is hampered. *See, e.g.,* Smiley et al. (1996) Truncation of the class II β-chain cytoplasmic domain influences the level of class II/invariant chain-derived peptide complexes, Proc. Natl. Acad. Sci. USA, 93:241-44. Truncation of Class II molecules seems to impair cAMP production. It has been postulated that deletion of the cytoplasmic tail of MHC II affects intracellular trafficking, thus preventing the complex from coming across relevant antigens in the endocytic pathway. Smiley et al. *(supra)* demonstrated that truncation of class II molecules at the cytoplasmic domain reduces the number of CLIP/class II complexes, postulating that this affects the ability of CLIP to effectively regulate antigen presentation.

It has been hypothesized that, since MHC II clustering is important for T cell receptor (TCR) triggering, if MHC II molecules truncated at the cytoplasmic domain were prevented from binding cytoskeleton and thus aggregating, antigen presentation to T cells would be affected. Ostrand-Rosenberg et al. (1991) Abrogation of Tumorigenicity by MHC Class II Antigen Expression Requires the Cytoplasmic Domain of the Class II Molecule, J. Immunol. 147:2419-22. In fact, it was recently shown that HLA-DR truncated at the cytoplasmic domain failed to associate with the cytoskeleton following oligomerization. El Fakhy et al. (2004) Delineation of the HLA-DR Region and the Residues Involved in the Association with the Cytoskeleton, J. Biol. Chem. 279:18472-80. Importantly, actin cytoskeleton is a site of localized signal transduction activity, which can affect antigen presentation. In addition to association with cytoskeleton, recent studies have also shown that up to 20% of all HLA-DR molecules constitutively reside in the lipid rafts of APCs, which are microdomains rich in cholesterol and glycosphingolipids, and that such localization is important for antigen presentation, immune synapse formation, and MHC II-mediated signaling. *See, e.g.,* Dolan et al. (2004) Invariant Chain and the MHC II Cytoplasmic Domains Regulate Localization of MHC Class II Molecules to Lipid Rafts in Tumor Cell-Based Vaccines, J. Immunol. 172:907-14. Dolan et al. suggested that truncation of cytoplasmic domain of MHC II reduces constitutive localization of MHC II to lipid rafts.

In addition, the cytoplasmic domain of MHC II, in particular the β chain, contains a leucine residue that is subject to ubiquitination by ubiquitin ligase, membrane-associated RING-CH I (MARCH I), which controls endocytic trafficking, internalization, and degradation of MHC II; and it has been shown that MARCH-mediated ubiquitination ceases upon dendritic cell maturation resulting in increased levels of MHC II at the plasma membrane. Shin et al. (2006) Surface expression of MHC class II in dendritic cells is controlled by regulated ubiquitination, Nature 444:115-18; De Gassart et al. (2008) MHC class II stabilization at the surface of human dendritic cells is the result of maturation-dependent MARCH I down-regulation, Proc. Natl. Acad. Sci. USA 105:3491-96.

Transmembrane domains of α and β chains of MHC II interact with each other and this interaction is important for proper assembly of class II MHC complex. Cosson and Bonifacino (1992) Role of Transmembrane Domain Interactions in the Assembly of Class II MHC Molecules, Nature 258:659-62. In fact, MHC II molecules in which the transmembrane domains of the α and β chains were replaced by the α chain of IL-2 receptor were retained in the ER and were barely detectable at the cell surface. *Id.* Through mutagenesis studies, conserved Gly residues at the α and β transmembrane domains were found to be responsible for MHC II assembly at the cell surface. *Id.* Thus, both transmembrane and cytoplasmic domains are crucial for the proper function of the MHC II complex.

Disclosed herein is a genetically modified mouse that comprises in its genome a nucleotide sequence encoding a human or humanized MHC II complex, e.g., a human or humanized MHC II α and/or β polypeptide(s). The mouse comprises in its genome a nucleotide sequence that encodes an MHC II complex that is partially human and partially mouse, e.g., a mouse that expresses a chimeric human/mouse MHC II complex (e.g., a mouse that expresses chimeric human/mouse MHC II α and β polypeptides). In one example, the mouse only expresses the human or humanized MHC II complex, e.g., a chimeric human/mouse MHC II complex, and does not express an endogenous mouse MHC II complex from an endogenous MHC II locus. In some examples, the mouse is incapable of expressing any endogenous mouse MHC II complex from an endogenous MHC II locus, but only expresses the human or humanized MHC II complex. In other examples, the mouse retains a nucleotide sequence encoding a functional endogenous mouse MHC II polypeptide. In various examples, the genetically modified mouse comprises in its germline a nucleotide sequence encoding a human or humanized MHC II complex, e.g., a human or humanized MHC II α and/or β polypeptide(s).

In one example, disclosed herein is a mouse comprising in its genome, e.g., at an endogenous mouse MHC II locus, a nucleotide sequence encoding a human MHC II polypeptide. In another example, disclosed herein is a mouse comprising in its genome, e.g., at an endogenous MHC II locus, a nucleotide sequence encoding a chimeric human/mouse MHC II polypeptide. Thus, also provided herein is a mouse that comprises in its genome, e.g., at an endogenous mouse MHC II locus, a nucleotide sequence(s) encoding a human or a chimeric human/mouse MHC II complex.

In one example, a chimeric human/mouse MHC II complex is provided. In one example, the chimeric human/mouse MHC II complex comprises a chimeric human/mouse MHC II α polypeptide and a chimeric human/mouse MHC II β polypeptide. In one example, a human portion of the chimeric MHC II α polypeptide and/or a human portion of the chimeric MHC II β polypeptide comprises a peptide-binding domain of a human MHC II α polypeptide and/or human MHC II β polypeptide, respectively. In one example, a human portion of the chimeric MHC II α and/or β polypeptide comprises an extracellular domain of a human MHC II α and/or β polypeptide, respectively. In one example, a human portion of the chimeric MHC II α polypeptide comprises α1 domain of a human MHC II α polypeptide; in another embodiment, a human portion of the chimeric MHC II α polypeptide comprises α1 and α2 domains of a human MHC II α polypeptide. In an additional example, a human portion of the chimeric MHC II β polypeptide comprises β1 domain of a human MHC II β polypeptide; in another example, a human portion of the chimeric MHC II β polypeptide comprises β1 and β2 domains of a human MHC II β polypeptide.

The human portion of the MHC II α and β polypeptides described herein may be encoded by any of HLA-DP, -DQ, and -DR loci. A list of commonly used HLA antigens and alleles is described in Shankarkumar et al. ((2004) The Human Leukocyte Antigen (HLA) System, Int. J. Hum. Genet. 4(2):91-103). Shankarkumar et al. also present a brief explanation of HLA nomenclature used in the art. Additional information regarding HLA nomenclature and various HLA alleles can be found in Holdsworth et al. (2009) The HLA dictionary 2008: a summary of HLA-A, -B, -C, -DRB1/3/4/5, and DQB1 alleles and their association with serologically defined HLA-A, -B, -C, -DR, and -DQ antigens, Tissue Antigens 73:95-170, and a recent update by Marsh et al. (2010) Nomenclature for factors of the HLA system, 2010, Tissue Antigens 75:291-455. Thus, the human or humanized MHC II polypeptide may be derived from any functional human HLA molecules described therein.

The human portions of the humanized MHC II complex described herein are derived from human HLA-DR2. Typically, HLA-DR α chains are monomorphic, e.g., the α chain of HLA-DR complex is encoded by HLA-DRA gene (e.g., HLA-DRα1*01 gene). On the other hand, the HLA-DR β chain is polymorphic. HLA-DR2 comprises an α chain encoded by HLA-DRA gene and a β chain encoded by HLA-DRB1 gene (e.g., HLA-DRβ1*02 gene). HLA-DR2 is known to be associated with a number of diseases, e.g., Goodpasture syndrome, multiple sclerosis, etc. In one example, the HLA-DRA allele is HLA-DRα*01 allele, e.g., HLA-DRα*01:01:01:01. In another example, the HLA-DRB allele is HLA-DRβ1*04, e.g., HLA-DRβ1*04:01:01. In another example, the HLA-DRB allele is HLA- DRβ1*02, e.g., HLA-DRβ1*1501.

In another example, the human portions of the humanized MHC II complex described herein are derived from human HLA-DQ, e.g., HLA-DQ2 and HLA-DQ8. HLA-DQ2 comprises an α chain encoded by HLA-DQA gene (e.g., HLA-DQα1*05 gene). In one example, HLA-DQα1*05 gene is HLA-DQα1*0501. HLA-DQ2 also comprises a β chain encoded by HLA-DQB gene (e.g., HLA-DQβ1*02 gene). HLA-DQ8 comprises an α chain encoded by HLA-DQA gene (e.g., HLA-DQα1*0301 gene). HLA-DQ8 also comprises a β chain encoded by HLA-DQB gene (e.g., HLA-DQβ1*0302 gene). HLA-DQ2.5 and HLA-DQ8 alleles are known to be associated with such diseases as Celiac disease and type I diabetes.

Although the present Examples describe these particular HLA sequences; any suitable HLA-DR or HLA-DQ sequences are disclosed herein, e.g., polymorphic variants exhibited in human population, sequences with one or more conservative or non-conservative amino acid modifications, nucleic acid sequences differing from the sequences described herein due to the degeneracy of genetic code, etc.

HLA alleles known to be associated with common human diseases include, but are not limited to, HLA-DRB1*0401, -DRB1*0301, -DQA1*0501, -DQB1*0201, -DRB1*1501, - DRB1*1502, -DQB1*0602, -DQA1*0102, -DQA1*0201, -DQB1*0202, -DQA1*0501, and combinations thereof. For a summary of HLA allele/disease associations, see Bakker et al. (2006) A high-resolution HLA and SNP haplotype map for disease association studies in the extended human MHC, Nature Genetics 38:1166-72 and Supplementary Information.

A mouse portion of the chimeric human/mouse MHC II complex comprises transmembrane and/or cytoplasmic domains of an endogenous mouse MHC II complex. Thus, a mouse portion of the chimeric human/mouse MHC II α polypeptide may comprise transmembrane and/or cytoplasmic domains of an endogenous mouse MHC II α polypeptide. A mouse portion of the chimeric human/mouse MHC II β polypeptide may comprise transmembrane and/or cytoplasmic domains of an endogenous mouse MHC II β polypeptide. In one embodiment, the mouse portions of the chimeric α and β polypeptides are derived from a mouse H-2E protein. Thus, mouse portions of the chimeric α and β polypeptides may comprise transmembrane and cytoplasmic domains derived from a mouse H-2E protein. In another embodiment, the mouse portions of the chimeric α and β polypeptides are derived from a mouse H-2A protein. Thus, mouse portions of the chimeric α and β polypeptides may comprise transmembrane and cytoplasmic domains derived from a mouse H-2A protein. Although specific H-2E and H-2A sequences are contemplated in the Examples, any suitable sequences, e.g., polymorphic variants, conservative/non-conservative amino acid substitutions, etc., are encompassed herein.

In various aspects of the invention, the sequence(s) encoding a chimeric human/mouse MHC II complex are located at an endogenous mouse H-2A and/or H-2E locus. In one embodiment, this results in a replacement of an endogenous MHC II gene(s) or a portion thereof with a nucleotide sequence(s) encoding a human or humanized MHC II protein, e.g., a chimeric gene encoding a chimeric human/mouse MHC II protein described herein. Since the nucleotide sequences encoding MHC II α and β polypeptides are located in proximity to one another on the chromosome, a replacement can be designed to target the two genes either independently or together; both of these possibilities are encompassed herein. In one embodiment, the replacement comprises a replacement of an endogenous nucleotide sequence encoding an MHC II α and β polypeptides with a nucleotide sequence encoding a chimeric human/mouse MHC α polypeptide and a chimeric human/mouse MHC β polypeptide. In one aspect, the replacement comprises replacing nucleotide sequences representing one or more (e.g., two) endogenous MHC II genes. Thus, the mouse contains a chimeric human/mouse nucleotide sequence at an endogenous MHC II locus, and expresses a chimeric human/mouse MHC II protein from the endogenous mouse locus.

Thus, disclosed herein is a mouse comprising at an endogenous MHC II gene locus a first nucleotide sequence encoding a chimeric human/mouse MHC II α polypeptide and a second nucleotide sequence encoding a chimeric human/mouse MHC II β polypeptide, wherein a human portion of the chimeric human/mouse MHC II α polypeptide comprises a human MHC II α extracellular domain and a human portion of the chimeric human/mouse MHC II β polypeptide comprises a human MHC II β extracellular domain, and wherein the chimeric human/mouse MHC II α and MHC II β polypeptides form a functional MHC II complex on a surface of a cell.

A chimeric human/mouse polypeptide may be such that it comprises a human or a mouse leader (signal) sequence. In one example, the chimeric MHC II α polypeptide comprises a mouse leader sequence of an endogenous MHC II α polypeptide. In one example, the chimeric MHC II β polypeptide comprises a mosue leader sequence of an endogenous MHC II β polypeptide. In an alternative example, the chimeric MHC II α and/or MHC II β polypeptide comprises a non-human leader sequence of MHC II α and/or MHC II β polypeptide, respectively, from another non-human animal, e.g., another rodent or another mouse strain. Thus, the nucleotide sequence encoding the chimeric MHC II α and/or MHC II β polypeptide may be operably linked to a nucleotide sequence encoding a mouse MHC II α and/or MHC II β leader sequence, respectively. In yet another example, the chimeric MHC II α and/or MHC II β polypeptide comprises a human leader sequence of human MHC II α and/or human MHC II β polypeptide, respectively. In one example, the human leader sequence of the human MHC II α is a leader sequence of human HLA-DRA and the human leader sequence of the human MHC II β polypeptide is a leader sequence of human HLA-DRβ1*04. In another example, the human leader sequence of the human MHC II α is a leader sequence of human HLA-DRA and the human leader sequence of the human MHC II β polypeptide is a leader sequence of human HLA-DRβ1*02. In another example, the human leader sequence of the human MHC II α is a leader sequence of human HLA-DQα1 *05 and the human leader sequence of the human MHC II β polypeptide is a leader sequence of human HLA-DQβ1*02. In yet another example, the human leader sequence of the human MHC II α is a leader sequence of human HLA-DQα1*0301 and the human leader sequence of the human MHC II β polypeptide is a leader sequence of human HLA-DQβ1*0302.

A chimeric human/mouse MHC II α and/or MHC II β polypeptide may comprise in its human portion a complete or substantially complete extracellular domain of a human MHC II α and/or human MHC II β polypeptide, respectively. Thus, a human portion may comprise at least 80%, preferably at least 85%, more preferably at least 90%, e.g., 95% or more of the amino acids encoding an extracellular domain of a human MHC II α and/or human MHC II β polypeptide. In one example, substantially complete extracellular domain of the human MHC II α and/or human MHC II β polypeptide lacks a human leader sequence. In another example, the chimeric human/non-human MHC II α and/or the chimeric human/mouse MHC II β polypeptide comprises a human leader sequence.

Moreover, the chimeric MHC II α and/or MHC II β polypeptide may be operably linked to (e.g., may be expressed under the control of) endogenous mouse promoter and regulatory elements, e.g., mouse MHC II α and/or MHC II β regulatory elements, respectively. Such arrangement will facilitate proper expression of the chimeric MHC II polypeptides in the mouse, e.g., during immune response in the mouse.

The genetically modified non-human animal is a mouse. For the non-human animals where suitable genetically modifiable ES cells are not readily available, other methods are employed to make a non-human animal comprising the genetic modification. Such methods include, e.g., modifying a non-ES cell genome (e.g., a fibroblast or an induced pluripotent cell) and employing nuclear transfer to transfer the modified genome to a suitable cell, e.g., an oocyte, and gestating the modified cell (e.g., the modified oocyte) in a non-human animal under suitable conditions to form an embryo.

The non-human animal is a mouse. In one embodiment, the genetically modified mouse is from a member of the family Muridae.

In a specific embodiment, the non-human animal is a mouse of a C57BL strain selected from C57BL/A, C57BL/An, C57BL/GrFa, C57BL/KaLwN, C57BL/6, C57BL/6J, C57BL/6ByJ, C57BL/6NJ, C57BL/10, C57BL/10ScSn, C57BL/10Cr, and C57BL/Ola. In another embodiment, the mouse is a 129 strain selected from the group consisting of a strain that is 129P1, 129P2, 129P3, 129X1, 129S1 (e.g., 129S1/SV, 129S1/SvIm), 129S2, 129S4, 129S5, 129S9/SvEvH, 129S6 (129/SvEvTac), 129S7, 129S8, 129T1, 129T2 (see, *e.g.,* Festing et al. (1999) Revised nomenclature for strain 129 mice, Mammalian Genome 10:836, *see also,* Auerbach et al (2000) Establishment and Chimera Analysis of 129/SvEv- and C57BL/6-Derived Mouse Embryonic Stem Cell Lines). In a specific embodiment, the genetically modified mouse is a mix of an aforementioned 129 strain and an aforementioned C57BL/6 strain. In another specific embodiment, the mouse is a mix of aforementioned 129 strains, or a mix of aforementioned BL/6 strains. In a specific embodiment, the 129 strain of the mix is a 129S6 (129/SvEvTac) strain. In another embodiment, the mouse is a BALB strain, e.g., BALB/c strain. In yet another embodiment, the mouse is a mix of a BALB strain and another aforementioned strain.

Thus, in one example, the invention relates to a genetically modified mouse that comprises in its genome a nucleotide sequence encoding a chimeric human/mouse MHC II complex, e.g., chimeric human/mouse MHC II α and β polypeptides. In one embodiment, a human portion of the chimeric human/mouse MHC II α polypeptide comprises a human MHC II α peptide binding or extracellular domain and a human portion of the chimeric human/mouse MHC II β polypeptide comprises a human MHC II β peptide binding or extracellular domain. In some embodiments, the mouse does not express a peptide binding or an extracellular domain of endogenous mouse α and/or β polypeptide from an endogenous mouse locus (e.g., H-2A and/or H-2E locus). In some embodiments, the mouse does not express functional peptide binding or extracellular domains of endogenous mouse MHC II polypeptides from endogenous mouse MHC II locus. In some embodiments, the mouse comprises a genome that lacks a gene that encodes a functional MHC class II molecule comprising an H-2Ab1, H-2Aa, H-2Eb1, H-2Eb2, H-2Ea, and a combination thereof. The peptide-binding domain of the human MHC II α polypeptide may comprise α1 domain and the peptide-binding domain of the human MHC II β polypeptide may comprise a β1 domain; thus, the peptide-binding domain of the chimeric MHC II complex may comprise human α1 and β1 domains. The extracellular domain of the human MHC II α polypeptide may comprise α1 and α2 domains and the extracellular domain of the human MHC II β polypeptide may comprise β1 and β2 domains; thus, the extracellular domain of the chimeric MHC II complex may comprise human α1, α2, β1 and β2 domains. In one embodiment, the mouse portion of the chimeric MHC II complex comprises transmembrane and cytosolic domains of mouse MHC II, e.g. mouse H-2E or H-2A (e.g., transmembrane and cytosolic domains of mouse H-2E α and β chains or mouse H-2A α and β chains).

In another embodiment, a genetically modified mouse is provided, wherein the mouse comprises at an endogenous mouse MHC II locus a first nucleotide sequence encoding a chimeric human/mouse MHC II α polypeptide and a second nucleotide sequence encoding a chimeric human/mouse MHC II β polypeptide, wherein a human portion of the chimeric MHC II α polypeptide comprises an extracellular domain derived from an α polypeptide of a human HLA-DR2 protein and a human portion of the chimeric MHC II β polypeptide comprises an extracellular domain derived from a β polypeptide of a human HLA-DR2 protein, wherein a mouse portion of the chimeric MHC II α polypeptide comprises transmembrane and cytoplasmic domains of a mouse H-2E α chain and a mouse portion of the chimeric MHC II β polypeptide comprises transmembrane and cytoplasmic domains of a mouse H-2E β chain, and wherein the mouse expresses a functional chimeric HLA-DR2/H-2E MHC II complex. In this embodiment the chimeric HLA-DR2/H-2E MHC II complex comprises an MHC II α chain that includes α1, and α2 domains derived from HLA-DR2 protein (HLA-DRA α1, and α2 domains) and transmembrane and cytoplasmic domains from a mouse H-2E α chain, as well as an MHC II β chain that includes β1 and β2 domains derived from HLA-DR2 (e.g. HLA-DRβ1 *02 β1 and β2 domains) and transmembrane and cytoplasmic domains from mouse H-2E β chain.

In another embodiment, a genetically modified mouse is provided, wherein the mouse comprises at an endogenous mouse MHC II locus a first nucleotide sequence encoding a chimeric human/mouse MHC II α polypeptide and a second nucleotide sequence encoding a chimeric human/mouse MHC II β polypeptide, wherein a human portion of the chimeric MHC II α polypeptide comprises an extracellular domain derived from an α polypeptide of a human HLA-DQ2 protein and a human portion of the chimeric MHC II β polypeptide comprises an extracellular domain derived from a β polypeptide of a human HLA-DQ2 protein, wherein a mouse portion of the chimeric MHC II α polypeptide comprises transmembrane and cytoplasmic domains of a mouse H-2A α chain and a mouse portion of the chimeric MHC II β polypeptide comprises transmembrane and cytoplasmic domains of a mouse H-2A β chain, and wherein the mouse expresses a functional chimeric HLA-DQ2/H-2A MHC II complex. In this embodiment the chimeric HLA-DQ2/H-2A MHC II complex comprises an MHC II α chain that includes α1, and α2 domains derived from HLA-DQ2 protein (e.g. HLA- DQα1*05 α1, and α2 domains) and transmembrane and cytoplasmic domains from a mouse H-2A α chain, as well as an MHC II β chain that includes β1 and β2 domains derived from HLA-DQ2 (e.g. HLA-DQβ1 *02 β1 and β2 domains) and transmembrane and cytoplasmic domains from mouse H-2A β chain.

In one aspect, the mouse does not express functional endogenous H-2A and H-2E polypeptides from their endogenous mouse loci (e.g., the mouse does not express H-2Ab1, H-2Aa, H-2Eb1, H-2Eb2, and H-2Ea polypeptides). In various embodiments, expression of the first and second nucleotide sequences is under the control of respective endogenous mouse promoters and regulatory elements (e.g., the first and second nucleotide sequences are operably linked to endogenous promoters and regulatory elements). In various embodiments of the invention, the first and the second nucleotide sequences are located on the same chromosome. In some aspects, the mouse comprises two copies of the chimeric MHC II locus containing the first and the second nucleotide sequences, while in other aspects, the mouse comprises one copy of the MHC II locus containing the first and the second nucleotide sequences. Thus, the mouse may be homozygous or heterozygous for the chimeric MHC II locus containing the first and the second nucleotide sequences. In various embodiments, the first and the second nucleotide sequences are comprised in the germline of the mouse.

As described herein, a mouse is provided that comprises a chimeric MHC II locus at an endogenous mouse MHC II locus, e.g., via replacement of endogenous mouse H-2A and H-2E genes. The various domains of the chimeric locus are linked in such a fashion that the locus expresses a functional chimeric human/mouse MHC II complex.

A mouse that expresses a functional chimeric MHC II protein from a chimeric MHC II locus as described herein displays the chimeric protein on a cell surface. In one embodiment, the mouse expresses the chimeric MHC II protein on a cell surface in a cellular distribution that is the same as observed in a human. In one aspect, the cell displays a peptide fragment (antigen fragment) bound to a human HLA-DR2extracellular portion of the chimeric MHC II protein.

In various examples, a cell displaying the chimeric MHC II protein (e.g., HLA-DR2/H-2E protein) is an antigen-presenting cell (APC) e.g., a macrophage, a dendritic cell, or a B cell. In some embodiments, the peptide fragment presented by the chimeric protein is derived from a tumor. In other embodiments, the peptide fragment presented by the chimeric MHC II protein is derived from a pathogen, e.g., a bacterium, a virus, or a parasite.

The chimeric MHC II protein described herein may interact with other proteins on the surface of the same cell or a second cell. In some examples, the chimeric MHC II protein interacts with endogenous non-human proteins on the surface of said cell. The chimeric MHC II protein may also interact with human or humanized proteins on the surface of the same cell or a second cell. In some examples, the second cell is a T cell, and the chimeric MHC II protein interacts with T cell receptor (TCR) and its co-receptor CD4. In some embodiments, the T cell is an endogenous mouse T cell. In other embodiments, the T cell is a human T cell. In some embodiments, the TCR is a human or humanized TCR. In additional embodiments, the CD4 is a human or humanized CD4. In other embodiments, either one or both of TCR and CD4 are mouse.

In one embodiment, a genetically modified mouse as described herein is provided that does not develop tumors at a higher rate than a wild-type mouse that lacks a chimeric MHC II gene. In some embodiments, the mouse does not develop hematological malignancies, e.g., various T and B cell lymphomas, leukemias, composite lymphomas (e.g., Hodgkin's lymphoma), at a higher rate than the wild-type animal.

In addition to a genetically engineered mouse, a mouse embryo is also disclosed, wherein the embryo comprises a donor ES cell that is derived from a mouse as described herein. In one example, the embryo comprises an ES donor cell that comprAlso disclosed is a tissue, wherein the tissue is derived from a mouse as described herein, and expresses the chimeric MHC II protein (e.g., HLA-DR2/H-2Eprotein).

In addition, a mouse cell isolated from a mouse as claimed is provided. In one embodiment, the cell is an ES cell. In one embodiment, the cell is an antigen-presenting cell, e.g., dendritic cell, macrophage, B cell. In one embodiment, the cell is an immune cell. In one embodiment, the immune cell is a lymphocyte.

Also disclosed is a mouse cell comprising a chromosome or fragment thereof of a mouse as described herein. In one embodiment, the mouse cell comprises a nucleus of a mouse as described herein. In one example, the mouse cell comprises the chromosome or fragment thereof as the result of a nuclear transfer.

**In** one example, a mouse induced pluripotent cell comprising gene encoding a chimeric MHC II protein (e.g., HLA-DR2/H-2Eprotein) as described herein is disclosed. In one example, the induced pluripotent cell is derived from a mouse as described herein.

In one example, a hybridoma or quadroma is disclosed, derived from a cell of a mouse as described herein.

In one example, an *in vitro* preparation is disclosed that comprises a first cell that bears a chimeric human/mouse MHC II surface protein that comprises a bound peptide to form a chimeric human/mouse MHC II/peptide complex, and a second cell that binds the chimeric human/mouse MHC II/peptide complex. In one example, the second cell comprises a human or humanized T-cell receptor, and in one example further comprises a human or humanized CD4. In one example, the second cell is a mouse cell comprising a human or humanized T-cell receptor and a human or humanized CD4 protein. In one example, the second cell is a human cell.

Also provided is a method for making a genetically engineered mouse described herein. The method for making a genetically engineered mouse results in a mouse whose genome comprises a nucleotide sequence encoding a chimeric MHC II protein (e.g., chimeric MHC II α and β polypeptides). In one embodiment, the method results in a genetically engineered mouse, whose genome comprises at an endogenous MHC II locus a nucleotide sequence encoding a chimeric human/mouse MHC II protein, wherein a human portion of the chimeric MHC II protein comprises an extracellular domain of a human HLA-DR2 and a mouse portion comprises transmembrane and cytoplasmic domains of a mouse H-2E. In some embodiments, the method utilizes a targeting construct made using VELOCIGENE^{®} technology, introducing the construct into ES cells, and introducing targeted ES cell clones into a mouse embryo using VELOCIMOUSE^{®} technology, as described in the Examples. In one embodiment, the ES cells are a mix of 129 and C57BL/6 mouse strains; in one embodiment, the ES cells are a mix of BALB/c and 129 mouse strains.

A nucleotide construct used for generating genetically engineered mice described herein is also disclosed. In one example, the nucleotide construct comprises: 5' and 3' mouse homology arms, a DNA fragment comprising human HLA-DR α and β chain sequences, and a selection cassette flanked by recombination sites. In one example, the human HLA-DR α and β chain sequences are genomic sequences that comprise introns and exons of human HLA-DR α and β chain genes. In one example, the mouse homology arms are homologous to mouse MHC II genomic sequence.

In one example, the human HLA-DR α chain sequence comprises an α1 and α2 domain coding sequence. In a specific example, it comprises, from 5' to 3': α1 exon (exon 2), α1/α2 intron (intron 2), and α2 exon (exon 3). In one example, the human HLA-DR β chain sequence comprises a β1 and β2 domain coding sequence. In a specific example, it comprises, from 5' to 3': β1 exon (exon 2), β1/β2 intron (intron 2), and β2 exon (exon 3).

Similarly, also disclosed herein are nucleotide constructs for generating genetically engineered mice comprising human HLA-DQ α and β chain sequences (e.g., human HLA-DQ α1 and α2 domain coding sequences and human HLA-DQ β1 and β2 domain coding sequences). Specific examples of such constructs are presented in FIG. 10.

A selection cassette is a nucleotide sequence inserted into a targeting construct to facilitate selection of cells (e.g., ES cells) that have integrated the construct of interest. A number of suitable selection cassettes are known in the art. Commonly, a selection cassette enables positive selection in the presence of a particular antibiotic (e.g., Neo, Hyg, Pur, CM, SPEC, etc.). In addition, a selection cassette may be flanked by recombination sites, which allow deletion of the selection cassette upon treatment with recombinase enzymes. Commonly used recombination sites are *loxP* and *Frt,* recognized by Cre and Flp enzymes, respectively, but others are known in the art. A selection cassette may be located anywhere in the construct outside the coding region. In one embodiment, the selection cassette is located in the β chain intron, e.g., β2/transmembrane domain intron (intron 3).

In one example, 5' and 3' homology arms comprise genomic sequence at 5' and 3' locations of endogenous mouse MHC II locus. In one example, the 5' homology arm comprises genomic sequence upstream of mouse H-2Ab1 gene and the 3' homology arm comprises genomic sequence downstream of mouse H-2Ea gene. In this example, the construct allows replacement of both mouse H-2E and H-2A genes.

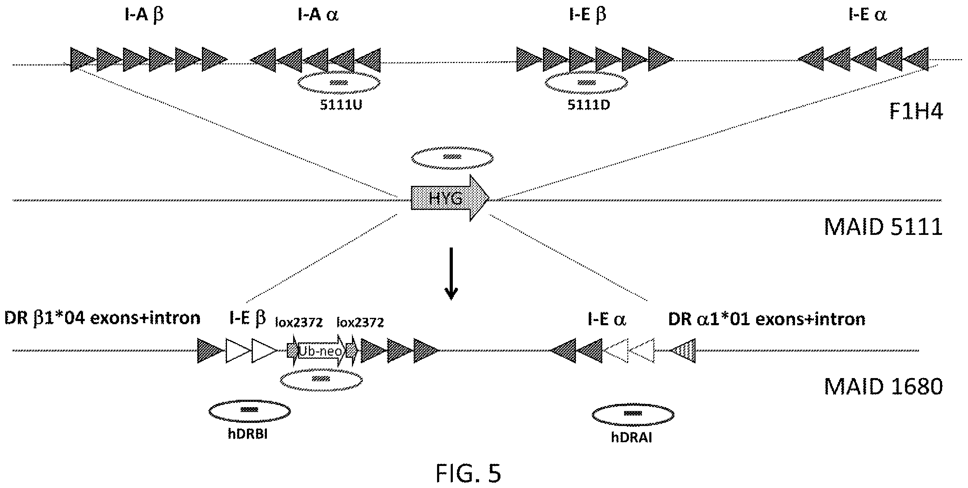

Thus, in one example, a nucleotide construct is disclosed comprising, from 5' to 3': a 5' homology arm containing mouse genomic sequence upstream of mouse H-2Ab1 gene, a first nucleotide sequence comprising a sequence encoding a chimeric human/mouse MHC II β chain, a second nucleotide sequence comprising a sequence encoding a chimeric human/mouse MHC II α chain, and a 3' homology arm containing mouse genomic sequence downstream of mouse H-2Ea gene. In a specific example, the first nucleotide sequence comprising a sequence encoding a chimeric human/mouse MHC II β chain comprises human β1 exon, β1/β2 intron, β2 exon, an a selection cassette flanked by recombination sites inserted in the intronic region between the human β2 exon sequence and the sequence of a mouse transmembrane domain exon. In a specific example, the second nucleotide sequence comprising a sequence encoding a chimeric human/mouse MHC II α chain comprises human α1 exon, α1/α2 intron, and human α2 exon. Exemplary constructs of the invention are depicted in FIGs. 5 and 10.

Upon completion of gene targeting, ES cells or genetically modified non-human animals are screened to confirm successful incorporation of exogenous nucleotide sequence of interest or expression of exogenous polypeptide. Numerous techniques are known to those skilled in the art, and include (but are not limited to) Southern blotting, long PCR, quantitative PCT (e.g., real-time PCR using TAQMAN^{®}), fluorescence *in situ* hybridization, Northern blotting, flow cytometry, Western analysis, immunocytochemistry, immunohistochemistry, etc. In one example, non-human animals (e.g., mice) bearing the genetic modification of interest can be identified by screening for loss of mouse allele and/or gain of human allele using a modification of allele assay described in Valenzuela et al. (2003) High-throughput engineering of the mouse genome coupled with high-resolution expression analysis, Nature Biotech. 21(6):652-659. Other assays that identify a specific nucleotide or amino acid sequence in the genetically modified animals are known to those skilled in the art.

Disclosed herein is a method of modifying an MHC II locus of a mouse to express a chimeric human/mouse MHC II complex described herein. In one example, the invention provides a method of modifying an MHC II locus of a mouse to express a chimeric human/mouse MHC II complex comprising replacing at the endogenous mouse MHC II locus a nucleotide sequence encoding a mouse MHC II complex with a nucleotide sequence encoding a chimeric human/mouse MHC II complex. In a specific example, the nucleotide sequence encoding the chimeric human/mouse MHC II complex comprises a first nucleotide sequences encoding an extracellular domain of a human MHC II α chain (e.g., HLA-DR or -DQ α chain) and transmembrane and cytoplasmic domains of a mouse MHC II α chain (e.g., H-2E or H-2A α chain) and a second nucleotide sequence encoding an extracellular domain of a human MHC II β chain (e.g., HLA-DR or -DQ β chain) and transmembrane and cytoplasmic domains of a mouse MHC II β chain (e.g., H-2E or H-2A β chain, e.g., H-2Eb1 or H-2Ab1 chain, respectively). In some embodiments, the modified mouse MHC II locus expresses a chimeric HLA-DR2/H-2E protein.

In one embodiment, a method for making a chimeric human HLA class II/mouse MHC class II molecule is provided, comprising expressing in a single cell a chimeric HLA-DR2/H-2Eprotein) from a nucleotide construct as described herein. In one example, the nucleotide construct is a viral vector; in a specific embodiment, the viral vector is a lentiviral vector. In one example, the cell is selected from a CHO, COS, 293, HeLa, and a retinal cell expressing a viral nucleic acid sequence (e.g., a PERC.6^{™} cell).

In embodiment, a cell that expresses a chimeric HLA-DR2/H-2E protein is provided. In one example, the cell comprises an expression vector comprising a chimeric MHC class II sequence as described herein. In one example, the cell is selected from CHO, COS, 293, HeLa, and a retinal cell expressing a viral nucleic acid sequence *(e.g.,* a PERC.6^{™} cell).

A chimeric MHC class II molecule made by a mouse as described herein is also provided, wherein the chimeric MHC class II molecule comprises α1, α2, β1, and β2 domains from a human HLA-DR2 and transmembrane and cytoplasmic domains from a mouse MHC II protein, e.g., mouse H-2E or H-2A protein. The chimeric MHC II complex comprising an extracellular domain of HLA-DR2 described herein may be detected by anti-HLA-DR antibodies. Thus, a cell displaying chimeric human/mouse MHC II polypeptide may be detected and/or selected using anti-HLA-DR antibody.

Although the Examples that follow describe a genetically engineered animal whose genome comprises a replacement of a nucleotide sequence encoding mouse H-2A and H-2E proteins with a nucleotide sequence encoding a chimeric human/mouse HLA-DR4/H-2E, HLA-DR2/H-2E, HLA-DQ2/H-2A, or HLA-DQ8/H-2A protein, one skilled in the art would understand that a similar strategy may be used to introduce chimeras comprising other human MHC II genes (other HLA-DR, HLA-DQ, or HLA-DP genes). In addition, introduction of multiple humanized MHC II molecules (e.g., chimeric HLA-DR/H-2E and HLA-DQ/H-2A) is also disclosed.

The replacement at multiple MHC II loci is provided. Thus, also provided herein is a mouse that comprises at an endogenous MHC II locus one or more, e.g., one, two, three, or four, nucleotide sequence(s) encoding a chimeric human/mouse MHC II complex(es). In one instance, each of the two mouse sister chromosomes 17 contains an MHC II locus that comprises H-2A and H-2E genes; thus, in one aspect, each sister chromosome may encode up to two chimeric human/mouse complexes at their endogenous genomic positions. Therefore, in one embodiment, a genetically modified mouse, may comprise up to four different nucleotide sequences encoding up to four chimeric human/mouse MHC II complexes at their endogenous MHC loci.

In one embodiment, provided herein is a mouse comprising at an endogenous MHC II locus one or more, e.g., one, two, three, or four, nucleotide sequence(s) encoding a chimeric human/mouse MHC II complex(es) wherein a human portion of the MHC II complex comprises an extracellular domain of a human MHC II complex (i.e., extracellular domains of human MHC II α and β polypeptides) and wherein a mouse portion of the chimeric MHC II complex comprises a transmembrane and cytoplasmic domain of a mouse MHC II complex (i.e., transmembrane and cytoplasmic domains of a mouse MHC II α and β polypeptides, e.g., H-2A and H-2E transmembrane and extracellular domains), and wherein the mouse expresses one or more, e.g., one, two, three, or four, chimeric human/mouse MHC II complex(es). The mouse MHC II is selected from H-2E and H-2A. The human MHC II is HLA-DR2.

Furthermore, provided herein is a method for generating a non-human animal, e.g., a mouse, comprising replacements at multiple endogenous MHC loci, e.g., a non-human, e.g., a mouse, comprising one or more, e.g., one, two, three, or four, nucleotide sequence(s) encoding a chimeric human/non-human, e.g., human/mouse, MHC II complex(es). Due to close linkage of the various MHC II loci on mouse chromosome 17, in some embodiments, the methods comprise successive replacements at the locus. In one embodiment, the method comprises replacing a nucleotide sequence encoding a first mouse MHC II complex with a nucleotide sequence encoding a first chimeric human/mouse MHC II complex in an ES cell, generating a mouse expressing the first chimeric MHC II complex, generating an ES cell from said mouse, replacing in said ES cell a nucleotide sequence encoding a second mouse MHC II complex with a nucleotide sequence encoding a second chimeric human/mouse MHC II complex, and generating a mouse expressing two chimeric human/mouse MHC II complexes. Alternatively, the method may comprise replacing a nucleotide sequence encoding a first mouse MHC II complex with a nucleotide sequence encoding a first chimeric human/mouse MHC II complex in an ES cell, followed by replacing in same ES cell a nucleotide sequence encoding a second mouse MHC II complex with a nucleotide sequence encoding a second chimeric human/mouse MHC II complex, and generating a mouse expressing two chimeric human/mouse MHC II complexes. A mouse comprising a nucleotide sequence encoding a third or fourth chimeric human/mouse MHC II complex can be generated by, e.g., breeding. One skilled in the art would understand that a mouse comprising two or more chimeric MHC II complexes may also be generated by breeding rather than successive replacement; this animal may be heterozygous for all of the chimeric MHC II sequences (e.g., a mouse comprising a different chimeric gene on each of its sister chromosomes will be heterozygous for the two MHC genes, etc.). Alternatively to successive replacements at the locus, a mouse comprising more than one, e.g., two, nucleotide sequences encoding chimeric human/mouse MHC II complexes may be generated by replacing a nucleotide sequence encoding more than one, e.g., two, MHC II complexes with an exogenous nucleotide sequence encoding more than one, e.g., two, chimeric human/mouse MHC II complexes (e.g., nucleotide sequence encoding both mouse H-2A and H-2E genes may be replaced by a nucleotide sequence encoding chimeric HLA-DR/H-2E and HLA-DQ/H-2A complexes).

### Use of Genetically Modified Animals

In various embodiments, the genetically modified non-human animals described herein make APCs with human or humanized MHC II on the cell surface and, as a result, present peptides derived from cytosolic proteins as epitopes for T cells in a human-like manner, because substantially all of the components of the complex are human or humanized. The genetically modified mice of the invention can be used to study the function of a human immune system in the humanized mouse; for identification of antigens and antigen epitopes that elicit immune response (e.g., T cell epitopes, e.g., unique human cancer epitopes), e.g., for use in vaccine development; for evaluation of vaccine candidates and other vaccine strategies; for studying human autoimmunity; for studying human infectious diseases; and otherwise for devising better therapeutic strategies based on human MHC expression.

MHC II complex binds peptides derived from extracellular proteins, e.g., extracellular bacterium, neighboring cells, or polypeptides bound by B cell receptors and internalized into a B cell. Once extracellular proteins enter endocytic pathway, they are degraded into peptides, and peptides are bound and presented by MHC II. Once a peptide presented by MHC II is recognized by CD4+ T cells, T cells are activated, proliferate, differentiate to various T helper subtypes (e.g., T_{H}1, T_{H}2), and lead to a number of events including activation of macrophage-mediated pathogen killing, B cell proliferation, and antibody production. Because of MHC II role in immune response, understanding of MHC II peptide presentation is important in the development of treatment for human pathologies. However, presentation of antigens in the context of mouse MHC II is only somewhat relevant to human disease, since human and mouse MHC complexes recognize antigens differently, e.g., a mouse MHC II may not recognize the same antigens or may present different epitopes than a human MHC II. Thus, the most relevant data for human pathologies is obtained through studying the presentation of antigen epitopes by human MHC II.

Thus, in various examples, the genetically engineered mice of the present invention are useful, among other things, for evaluating the capacity of an antigen to initiate an immune response in a human, and for generating a diversity of antigens and identifying a specific antigen that may be used in human vaccine development.

In one example, a method for determining antigenicity in a human of a peptide sequence is provided, comprising exposing a genetically modified mouse as described herein to a molecule comprising the peptide sequence, allowing the mouse to mount an immune response, and detecting in the mouse a cell that binds a sequence of the peptide presented by a humanized MHC II complex described herein.

In one example, a method for determining whether a peptide will provoke an immune response in a human is disclosed, comprising exposing a genetically modified mouse as described herein to the peptide, allowing the non-human mouse to mount an immune response, and detecting in the mouse a cell that binds a sequence of the peptide by a chimeric human/mouse MHC class II molecule as described herein. In one example, the mouse following exposure comprises an MHC class II-restricted CD4+ T cell that binds the peptide.

In one aspect, a method for identifying a human CD4+ T cell epitope is provided, comprising exposing a mouse as claimed to an antigen comprising a putative T cell epitope, allowing the mouse to mount an immune response, and identifying the epitope bound by the MHC class II-restricted CD4+ T cell.

In one example, a method is disclosed for identifying an antigen that generates a CD4+ T cell response in a human, comprising exposing a putative antigen to a mouse as described herein, allowing the mouse to generate an immune response, detecting a CD4+ T cell response that is specific for the antigen in the context of a human MHC II molecule (e.g., an HLA-DR or HLA-DQ molecule), and identifying the antigen bound by the human MHC II-restricted molecule (e.g., human HLA-DR or HLA-DQ restricted molecule).

In one example, the antigen comprises a bacterial protein. In one embodiment, the antigen comprises a human tumor cell antigen. In one embodiment, the antigen comprises a putative vaccine for use in a human, or another biopharmaceutical. In one embodiment, the antigen comprises a human epitope that generates antibodies in a human. In yet another embodiment, an antigen comprises a yeast or fungal cell antigen. In yet another embodiment, an antigen is derived from a human parasite.

In one aspect, a method is disclosed for determining whether a putative antigen contains an epitope that upon exposure to a human immune system will generate an HLA-DR-restricted immune response (e.g., HLA-DR2-restricted response), comprising exposing a mouse as described herein to the putative antigen and measuring an antigen-specific HLA-DR-restricted (e.g., HLA-DR2- or HLA-DR4-restricted) immune response in the mouse. In another example, a method is provided for determining wherein a putative antigen contains an epitope that upon exposure to a human immune system will generate an HLA-DQ-restricted (e.g., HLA-DQ2- or HLA-DQ8-restricted) immune response.

Also disclosed is a method of generating antibodies to an antigen, e.g., an antigen derived from bacterium, parasite, etc., presented in the context of a human MHC II complex, comprising exposing a mouse described herein to an antigen, allowing a mouse to mount an immune response, wherein the immune response comprises antibody production, and isolating an antibody that recognizes the antigen presented in the context of human MHC II complex. In one example, in order to generate antibodies to the peptide-MHC II, the MHC II humanized mouse is immunized with a peptide-MHC II immunogen.

In one example, a method for identifying a T cell receptor variable domain that recognizes an antigen presented in the context of MHC II (e.g., human tumor antigen, a vaccine, etc.) is disclosed, comprising exposing a mouse comprising a humanized MHC II complex described herein to the antigen, allowing the mouse to generate an immune response, and isolating from the mouse a nucleic acid sequence encoding a variable domain of a T cell receptor that binds MHC II-restricted antigen. In one embodiment, the antigen is presented in the context of a humanized MHC II (e.g., human HLA II ectodomain/mouse MHC II transmembrane and/or cytoplasmic domain).

The consequence of interaction between a T cell and an APC displaying a peptide in the context of MHC II (e.g., human HLA II ectodomain/mouse MHC II transmembrane and/or cytoplasmic domain) can be measured by a number of techniques known in the art, e.g., T cell proliferation assays, cytokine release assays, etc.

In addition to the ability to identify antigens and their T cell epitopes from pathogens or neoplasms, the genetically modified mice of the invention can be used to identify autoantigens of relevance to human autoimmune disease, and otherwise study human autoimmune disease progression. It is known that polymorphisms within the HLA loci play a role in predisposition to human autoimmune disease. In fact, specific polymorphisms in HLA-DR and HLA-DQ loci have been identified that correlate with development of rheumatoid arthritis, type I diabetes, Hashimoto's thyroiditis, multiple sclerosis, myasthenia gravis, Graves' disease, systemic lupus erythematosus, celiac disease, Crohn's disease, ulcerative colitis, and other autoimmune disorders. See, e.g., Wong and Wen (2004) What can the HLA transgenic mouse tell us about autoimmune diabetes?, Diabetologia 47:1476-87; Taneja and David (1998) HLA Transgenic Mice as Humanized Mouse Models of Disease and Immunity, J. Clin. Invest. 101:921-26; Bakker et al. (2006), *supra;* and International MHC and Autoimmunity Genetics Network (2009) Mapping of multiple susceptibility variants within the MHC region for 7 immune-mediated diseases, Proc. Natl. Acad. Sci. USA 106:18680-85.

Thus, the methods of making a humanized MHC II complex mice described herein can be used to introduce MHC II molecules thought to be associated with specific human autoimmune diseases, and progression of human autoimmune disease can be studied. In addition, mice described herein can be used to develop animal models of human autoimmune disease. Mice according to the invention carrying humanized MHC II proteins described herein can be used to identify potential autoantigens, to map epitopes involved in disease progression, and to design strategies for autoimmune disease modulation.

In addition, the genetically modified micels described herein may be used in the study of human allergic response. As allergic responses appear to be associated with MHC II alleles, genetically modified mice described herein may be used to determine HLA restriction of allergen specific T cell response and to develop strategies to combat allergic response.

In addition, the genetically modified mice of the invention and the human or humanized HLA molecules expressed by the same can be used to test antibodies that block antigen presentation by human HLA molecules associated with human disease progression. Thus, disclosed herein is a method of determining whether an antibody is capable of blocking presentation of an antigen by an HLA molecule linked to a human disease, e.g., a human disease described above, comprising exposing a cell expressing a human or humanized HLA described herein to a test antibody and determining whether the test antibody is capable of blocking the presentation of the antigen by a human or humanized HLA to immune cells (e.g., to T cells), e.g., by measuring its ability to block human or humanized HLA-restricted immune response. In one example, the method is conducted in a mouse expressing the human or humanized HLA, e.g., a mouse expressing disease-associated human or humanized HLA that serves as a disease model for the disease.

### EXAMPLES

The invention will be further illustrated by the following nonlimiting examples. These Examples are set forth to aid in the understanding of the invention but are not intended to, and should not be construed to, limit its scope in any way. The Examples do not include detailed descriptions of conventional methods that would be well known to those of ordinary skill in the art (molecular cloning techniques, etc.). Unless indicated otherwise, parts are parts by weight, molecular weight is average molecular weight, temperature is indicated in Celsius, and pressure is at or near atmospheric.

### Example 1. Deletion of the Endogenous MHC class II H-2A and H-2E Loci

The targeting vector for introducing a deletion of the endogenous MHC class II H-2Ab1, H-2Aa, H-2Eb1, H-2Eb2, and H-2Ea genes was made using VELOCIGENE^{®} genetic engineering technology (see, *e.g.,* US Pat. No. 6,586,251 and Valenzuela et al., *supra).* Bacterial Artificial Chromosome (BAC) RP23-458i22 (Invitrogen) DNA was modified to delete the endogenous MHC class II genes H-2Ab1, H-2Aa, H-2Eb1, H-2Eb2, and H-2Ea.

Briefly, upstream and downstream homology arms were derived by PCR of mouse BAC DNA from locations 5' of the H-2Ab1 gene and 3' of the H-2Ea gene, respectively. As depicted in FIG. 5, these homology arms were used to make a cassette that deleted ~79 kb of RP23-458i22 comprising genes H-2Ab1, H-2Aa, H-2Eb1, H-2Eb2, and H-2Ea of the MHC class II locus by bacterial homologous recombination (BHR). This region was replaced with a hygromycin cassette flanked by lox66 and lox71 sites. The final targeting vector from 5' to 3' included a 34 kb homology arm comprising mouse genomic sequence 5' to the H-2Ab1 gene of the endogenous MHC class II locus, a 5' lox66 site, a hygromycin cassette, a 3' lox71 site and a 63 kb homology arm comprising mouse genomic sequence 3' to the H-2Ea gene of the endogenous MHC class II locus (MAID 5111, see FIG. 5).

The BAC DNA targeting vector (described above) was used to electroporate mouse ES cells to create modified ES cells comprising a deletion of the endogenous MHC class II locus. Positive ES cells containing a deleted endogenous MHC class II locus were identified by the quantitative PCR assay using TAQMAN^{™} probes (Lie and Petropoulos (1998) Curr. Opin. Biotechnology 9:43-48). The upstream region of the deleted locus was confirmed by PCR using primers 5111U F (CAGAACGCCAGGCTGTAAC; SEQ ID NO:1) and 5111U R (GGAGAGCAGGGTCAGTCAAC; SEQ ID NO:2) and probe 5111U P (CACCGCCACTCACAGCTCCTTACA; SEQ ID NO:3), whereas the downstream region of the deleted locus was confirmed using primers 5111D F (GTGGGCACCATCTTCATCATTC; SEQ ID NO:4) and 5111D R (CTTCCTTTCCAGGGTGTGACTC; SEQ ID NO:5) and probe 5111D P (AGGCCTGCGATCAGGTGGCACCT; SEQ ID NO:6). The presence of the hygromycin cassette from the targeting vector was confirmed using primers HYGF (TGCGGCCGATCTTAGCC; SEQ ID NO:7) and HYGR (TTGACCGATTCCTTGCGG; SEQ ID NO:8) and probe HYGP (ACGAGCGGGTTCGGCCCATTC; SEQ ID NO:9). The nucleotide sequence across the upstream deletion point (SEQ ID NO:10) included the following, which indicates endogenous mouse sequence upstream of the deletion point (contained within the parentheses below) linked contiguously to cassette sequence present at the deletion point: (TTTGTAAACA AAGTCTACCC AGAGACAGAT GACAGACTTC AGCTCCAATG CTGATTGGTT CCTCACTTGG GACCAACCCT) CTCGAGTACC GTTCGTATAA TGTATGCTAT ACGAAGTTAT ATGCATCCGG GTAGGGGAGG. The nucleotide sequence across the downstream deletion point (SEQ ID NO:11) included the following, which indicates cassette sequence contiguous with endogenous mouse sequence downstream of the deletion point (contained within the parentheses below): CCTCGACCTG CAGCCCTAGG ATAACTTCGT ATAATGTATG CTATACGAAC GGTAGAGCTC (CACAGGCATT TGGGTGGGCA GGGATGGACG GTGACTGGGA CAATCGGGAT GGAAGAGCAT AGAATGGGAG TTAGGGAAGA). Positive ES cell clones were then used to implant female mice using the VELOCIMOUSE^{®} method (described below) to generate a litter of pups containing a deletion of the endogenous MHC class II locus.

Targeted ES cells described above were used as donor ES cells and introduced into an 8-cell stage mouse embryo by the VELOCIMOUSE^{®} method (see, *e.g.,* US Pat. No. 7,294,754 and Poueymirou et al. (2007) F0 generation mice that are essentially fully derived from the donor gene-targeted ES cells allowing immediate phenotypic analyses, Nature Biotech. 25(1):91-99). Mice bearing a deletion of H-2Ab1, H-2Aa, H-2Eb1, H-2Eb2, and H-2Ea genes in the endogenous MHC class II locus were identified by genotyping using a modification of allele assay (Valenzuela *et al., supra)* that detected the presence of the hygromycin cassette and confirmed the absence of endogenous MHC class II sequences.

Mice bearing a deletion of H-2Ab1, H-2Aa, H-2Eb1, H-2Eb2, and H-2Ea genes in the endogenous MHC class II locus can be bred to a Cre deletor mouse strain (see, e.g., International Patent Application Publication No. WO 2009/114400) in order to remove any loxed hygromycin cassette introduced by the targeting vector that is not removed, e.g., at the ES cell stage or in the embryo. Optionally, the hygromycin cassette is retained in the mice.

### Example 2. Generation of Large Targeting Vector (LTVEC) Comprising Chimeric HLA-DR4/H-2E Gene

A targeting vector to introduce humanized MHC II sequences was designed as depicted in FIG. 4. Using VELOCIGENE^{®} genetic engineering technology, Bacterial Artificial Chromosome (BAC) RP23-458i22 DNA was modified in various steps to: (1) create a vector comprising a functional I-E α exon 1 from BALB/c H-2Ea gene (FIG. 4A); (2) create a vector comprising replacement of exons 2 and 3 of mouse I-E β gene with those of human DRβ1*04 and replacement of exons 2 and 3 of mouse I-E α with those of human DRα1*01 (FIGs. 4B); (3) create a vector carrying exons 2 and 3 of human DRβ1*04 amongst remaining mouse I-E β exons, and exons 2 and 3 of human DRα1*01 amongst remaining mouse I-E α exons including a functional I-E α exon 1 from BALB/c mouse (step (1) (FIG. 4C); and (4) remove a cryptic splice site in the vector generated in (3) (FIG. 4D).

Specifically, because in the C57Bl/6 mice, the I-E α gene is a pseudogene due to the presence of a non-functional exon 1, first, a vector comprising a functional I-E α exon 1 from BALB/c H-2Ea gene was created (FIG. 4A). RP23-458i22 BAC was modified by bacterial homologous recombination (1.BHR) to replace chloramphenicol resistance gene with that of spectromycin. The resultant vector was further modified by BHR to replace the entire I-A and I-E coding region with a neomycin cassette flanked by recombination sites (2.BHR). Another round of BHR (3. BHR) with the construct comprising an exon encoding BALB/c I-Eα leader (exon 1) and chloramphenicol gene flanked by PI-SceI and I-CeuI restriction sites resulted in a vector comprising a functional BALB/c H-2Ea exon 1.

Independently, in order to generate a vector comprising replacement of exons 2 and 3 of mouse I-E β gene with those of human DRβ1*04 and replacement of exons 2 and 3 of mouse I-E α with those of human DRα1*01, RP23-458i22 BAC was modified via several homologous recombination steps, 4. BHR - 8. BHR (FIG. 4B). The resultant nucleic acid sequence was flanked by PI-SceI/I-CeuI restriction sites to allow ligation into the construct carrying BALB/c I-Eα exon 1, mentioned above (FIG. 4C).

The sequence of the final construct depicted in FIG. 4C contained a cryptic splice site at the 3' end of the BALB/c intron. Several BHR steps (11. BHR - 12. BHR) followed by a deletion step were performed to obtain the final targeting vector (MAID 1680) that was used to electroporate into ES cells (FIG. 4D).

In detail, the final targeting vector (MAID 1680), from 5' to 3', was comprised of a 5' mouse homology arm consisting of ~26 kb of mouse genomic sequence ending just upstream of the H-2Ab1 gene of the endogenous MHC class II locus; an ~59 kb insert containing the humanized MHC II β chain gene (humanized H-2Eb1 gene) and humanized MHC II α chain gene (humanized H-2Ea gene) and a floxed neomycin cassette; and a 3' mouse homology arm consisting of ~57 kb of mouse genomic sequence beginning just downstream of the H-2Ea gene of the endogenous MHC class II locus. The nucleotide sequence across the junction between the 5' arm and the insert (SEQ ID NO:12) included the following: (TGCTGATTGG TTCCTCACTT GGGACCAACC C) TAAGCTTTA *TCTATGTCGG GTGCGGAGAA AGAGGTAATG AAATGGCACA* AGGAGATCAC ACACCCAAAC CAAACTCGCC, where the italicized sequence is a unique PI-SceI site, and mouse genomic sequence in the 5' homology arm is in parentheses. The nucleotide sequence across the junction between the insert and the 3' arm (SEQ ID NO: 13) included the following: CACATCAGTG AGGCTAGAAT AAATTAAAAT CGCTAATATG AAAATGGGG (ATTTGTACCT CTGAGTGTGA AGGCTGGGAA GACTGCTTTC AAGGGAC), where the mouse genomic sequence in the 3' homology arm is in parentheses.

Within the ~59 kb insert, the H-2Eb1 gene was modified as follows: a 5136 bp region of H-2Eb1, including the last 153 bp of intron1, exon 2, intron 2, exon 3, and the first 122 bp of intron 3, was replaced with the 3111 bp homologous region of human HLA-DRB1 *04, including the last 148 bp of intron 1, exon 2, intron 2, exon 3, and the first 132 bp of intron 3. At the junction between the human and mouse sequences of intron 3, a cassette consisting of a 5' lox2372 site, UbC promoter, neomycin resistance gene, and a 3' lox2372 site, was inserted. The resulting gene encoded a chimeric HLA-DRB1 *04/H-2Eb1 protein comprised of the mouse H-2Eb1 leader, the human β1 and β2 domains from DRB1*04, and the mouse transmembrane domain and cytoplasmic tail. The nucleotide sequence across the mouse/human junction in intron 1 (SEQ ID NO:14) included the following: (TCCATCACTT CACTGGGTAG CACAGCTGTA ACTGTCCAGC CTG) *GGTACCGAGC TCGGATCCAC TAGTAACGGC CGCCAGTGTG CTGGAATTC GCCCTTGATC GAGCTCCCTG* GGCTGCAGGT GGTGGGCGTT GCGGGTGGGG CCGGTTAA, where the italicized sequence is a multiple cloning site introduced during the cloning steps, and the mouse intron 1 sequences are in parentheses. The nucleotide sequence across the junction between the human intron 3 and neomycin cassette (SEQ ID NO:15) included the following: (ATCTCCATCA GAAGGGCACC GGT) *ATAACTT CGTATAAGGT ATCCTATACG AAGTTATATG* CATGGCCTCC GCGCCGGGTT, where the 5' lox2372 site is italicized, and human intron 3 sequence is in parentheses. The nucleotide sequence across the junction between the neomycin cassette and mouse intron 3 (SEQ ID NO:16) included the following: *ATAACTTCGT ATAAGGTATC CTATACGAAG TTATCTCGAG* (TGGCTTACAG GTAGGTGCGT GAAGCTTCTA CAAGCACAGT TGCCCCCTGG), where the 3' lox2372 site is italicized, and the mouse intron 3 sequence is in parentheses.

Also within the ~59 kb insert, the H-2Ea gene was modified as follows: a 1185 bp region of H-2Ea, including the last 101 bp of intron1, exon 2, intron 2, exon 3, and the first 66 bp of intron 3, was replaced with the 1189 bp homologous region of human HLA-DRA1*01, including the last 104 bp of intron 1, exon 2, intron 2, exon 3, and the first 66 bp of intron 3. As described above, because exon 1 of the C57BL/6 allele of H-2Ea contains a deletion which renders the gene nonfunctional, H-2Ea exon 1 and the remainder of intron 1 were replaced with the equivalent 2616 bp region from the BALB/c allele of H-2Ea, which is functional. The resulting gene encoded a chimeric H-2Ea/HLA-DRA1*01 protein comprised of the mouse H-2Ea leader from BALB/c, the human α1 and α2 domains from DRA1*01, and the mouse transmembrane domain and cytoplasmic tail. The nucleotide sequence across the mouse/human junction in intron 1 (SEQ ID NO:17) included the following: (CTGTTTCTTC CCTAACTCCC ATTCTATGCT CTTCCATCCC GA) *CCGCGGCCCA* ATCTCTCTCC ACTACTTCCT GCCTACATGT ATGTAGGT, where the italicized sequence is a restriction enzyme site introduced during the cloning steps, and the BALB/c intron 1 sequences are in parentheses. The nucleotide sequence across the human/mouse junction in intron 3 (SEQ ID NO:18) included the following: CAAGGTTTCC TCCTATGATG CTTGTGTGAA ACTCGGGGCC *GGCC* (AGCATTTAAC AGTACAGGGA TGGGAGCACA GCTCAC), where the italicized sequence is a restriction enzyme site introduced during the cloning steps, and the mouse intron 3 sequences are in parentheses. The nucleotide sequence across the C57BL/6-BALB/c junction 5' of exon 1 (SEQ ID NO:19) included the following: (GAAAGCAGTC TTCCCAGCCT TCACACTCAG AGGTACAAAT) CCCCATTTTC ATATTAGCGA TTTTAATTTA TTCTAGCCTC, where the C57BL/6-specific sequences are in parentheses. The nucleotide sequence across the BALB/c-C57BL/6 junction 3' of exon 1 (SEQ ID NO:20) included the following: TCTTCCCTAA CTCCCATTCT ATGCTCTTCC ATCCCGA *CCG CGG* (CCCAATC TCTCTCCACT ACTTCCTGCC TACATGTATG), where SacII restriction site is italicized, and C57BL/6 sequences are in parenthesis.

### Example 3. Generation of Genetically Modified HLA-DR4 Mice

Simplified diagrams of the strategy for generating humanized MHC II mice using the vector of Example 2 are presented in FIGs. 5 and 8.

Specifically, MAID1680 BAC DNA (described above) was used to electroporate MAID5111 ES cells to create modified ES cells comprising a replacement of the endogenous mouse I-A and I-E loci with a genomic fragment comprising a chimeric human DR4/mouse I-E locus. Positive ES cells containing deleted endogenous I-A and I-E loci replaced by a genomic fragment comprising a chimeric human DR4/mouse I-E locus were identified by a quantitative PCR assay using TAQMAN^{™} probes (Lie and Petropoulos, *supra).* The insertion of the human DRα sequences was confirmed by PCR using primers hDRA1F (CTGGCGGCTTGAAGAATTTGG; SEQ ID NO:21), hDRA1R (CATGATTTCCAGGTTGGCTTTGTC; SEQ ID NO:22), and probe hDRA1P (CGATTTGCCAGCTTTGAGGCTCAAGG; SEQ ID NO:23). The insertion of the human DRβ sequences was confirmed by PCR using primers hDRB1F (AGGCTTGGGTGCTCCACTTG; SEQ ID NO:24), hDRB1R (GACCCTGGTGATGCTGGAAAC; SEQ ID NO:25), and probe hDRB1P (CAGGTGTAAACCTCTCCACTCCGAGGA; SEQ ID NO:26).The loss of the hygromycin cassette from the targeting vector was confirmed with primers HYGF (TGCGGCCGATCTTAGCC; SEQ ID NO:7) and HYGR (TTGACCGATTCCTTGCGG; SEQ ID NO:8) and probe HYGP (ACGAGCGGGTTCGGCCCATTC; SEQ ID NO:9).

Positive ES cell clones were then used to implant female mice using the VELOCIMOUSE^{®} method *(supra)* to generate a litter of pups containing a replacement of the endogenous I-A and I-E loci with a chimeric human DR4/mouse I-E locus. Targeted ES cells described above were used as donor ES cells and introduced into an 8-cell stage mouse embryo by the VELOCIMOUSE^{®} method. Mice bearing a chimeric human DR4/mouse I-E locus were identified by genotyping using a modification of allele assay (Valenzuela et al., *supra)* that detected the presence of a chimeric human DR4/mouse I-E locus.

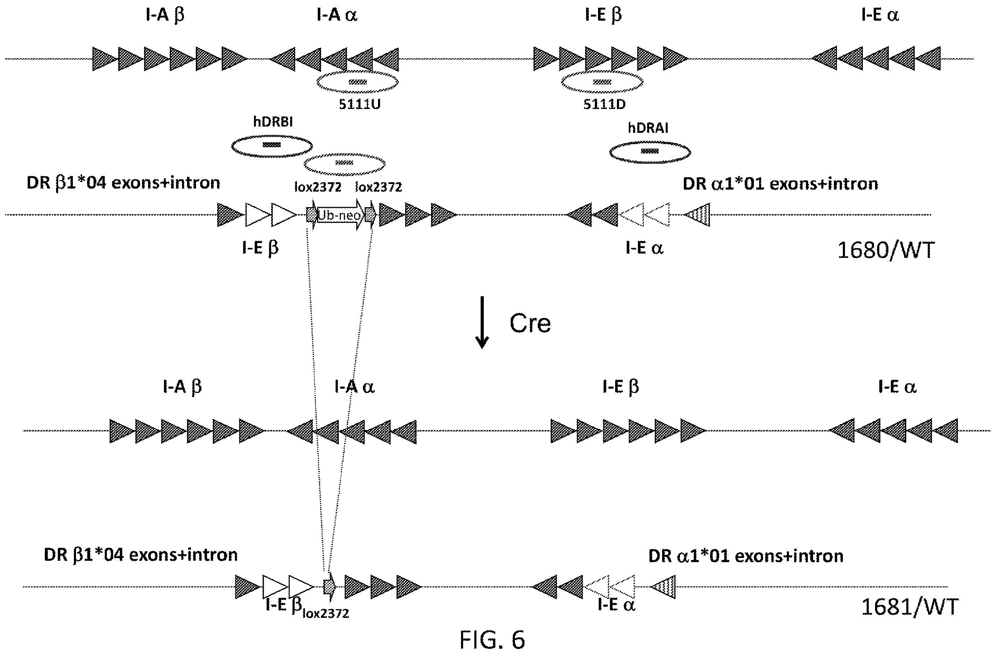

Mice bearing a chimeric human DR4/mouse I-E locus can be bred to a Cre deletor mouse strain (see, e.g., International Patent Application Publication No. WO 2009/114400) in order to remove any loxed neomycin cassette introduced by the targeting vector that is not removed, e.g., at the ES cell stage or in the embryo (See FIG. 6).

### Example 4. Expression of the Chimeric HLA-DR4/H-2E in Genetically Modified Mice

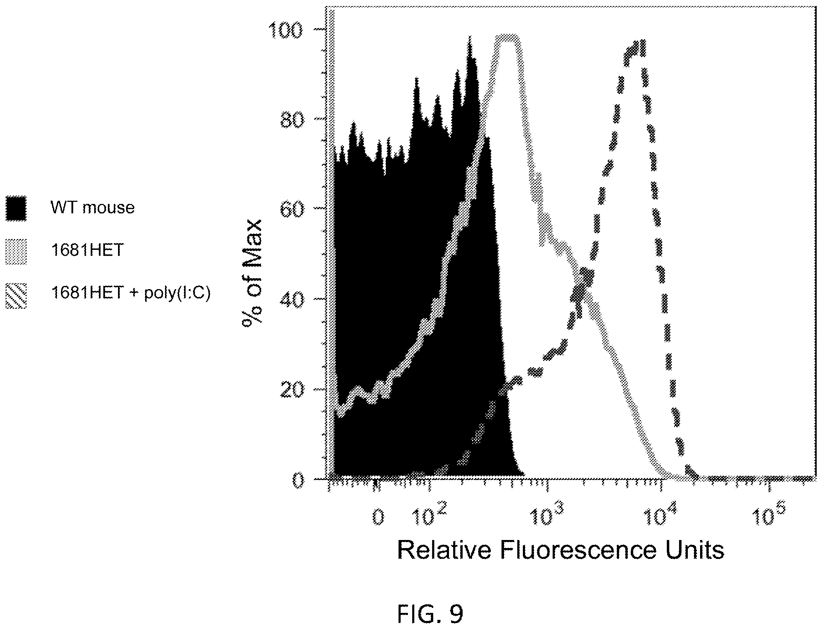

Spleens from WT or heterozygous humanized HLA-DR4 mice ("1681 HET") were perfused with Collagenase D (Roche Bioscience) and erythrocytes were lysed with ACK lysis buffer. Splenocytes were cultured for two days with 25 micrograms/mL poly(I:C) to stimulate the expression of MHC-II genes. Cell surface expression of human HLA-DR4 was analyzed by FACS using fluorochrome-conjugated anti-CD3 (17A2), anti-CD19 (1D3), anti-CD11c (N418), anti-F480 (BM8), anti-I-A/I-E (M15) and anti-HLADR (L243). Flow cytometry was performed using BD-LSRII. Expression of human HLA-DR4 was clearly detectable on the surface of CD19+ B cells and was significantly upregulated upon stimulation by toll-like receptor agonist poly(I:C) (see FIG. 9).

### Example 5. Genetically Modified HLA-DQ2.5, HLA-DQ8, and HLA-DR2 Mice

Mice harboring chimeric human HLA-DQ2.5/H-2A, HLA-DQ8/H-2A and HLA-DR2/H-2E were made using VELOCIGENE^{®} genetic engineering technology (see, *e.g.,* US Pat. No. 6,586,251 and Valenzuela et al., *supra).* LTVECs harboring humanized genes were generated by gene synthesis and modification of BACs by bacterial homologous recombination and digestion/ligation techniques.

Human HLA-DQ2.5, -DQ8.1, and DR2 α and β chain sequences were synthesized by Blue Heron Gene Synthesis Company (WA, USA) based on publically available gene sequences. Specifically, synthesized human HLA-DQ2.5 α (DQA1*05) and β (DQB1*02) chains and mouse BAC RP23-444J20 were modified and used to generate an LTVEC harboring chimeric HLA-DQ2.5/H-2A, depicted in FIG.10A. Similarly, synthesized human HLA-DQ8.1 α (DQA1*0301) and β (DQB1*0302) chains and mouse BAC RP23-444J20 were modified and used to generate an LTVEC harboring chimeric HLA-DQ8.1/H-2A, depicted in FIG. 10B. For HLA-DR2/H-2E, synthesized human HLA-DR2 β chain (DRB1*1501) was used to generate a vector comprising DRβ1*02(1501) exons and intron, and swapped using bacterial homologous recombination into the LTVEC comprising chimeric HLA-DR4/H-2E gene (MAID 1680 of Example 2 above). The resulting HLA-DR2/H-2E LTVEC is depicted in FIG. 10C. The various nucleotide sequence junctions of the resulting LTVECs (e.g., mouse/human sequence junctions, human/mouse sequence junctions, or junctions of mouse or human sequence with selection cassettes) are summarized below in Table 1 and listed in the Sequence Listing; their locations are indicated in the schematic diagram of FIG. 10. In Table 1 below, the mouse sequences are in regular font; the human sequences are in parentheses; the Lox sequences are italicized; and the restriction sites introduced during cloning steps and other vector-based sequences (e.g., multiple cloning sites, etc.) are bolded. The nucleotide sequence junctions for the humanized H-2Ea in HLA-DR2/H-2E are the same as previously described for HLA-DR4/H-2E in Example 2 above (SEQ ID NOs: 17-20).

**Table 1: Junctions of LTVECs for HLA-DR2/H-2E, HLA-DQ2.5/H-2A, and HLA-DQ8/H-2A Mice**

| **SEQ ID NO** | **Nucleotide Sequence** |
|---|---|
| **27** | GAAGAGACTGCCCGGCCCGAGGCCCCGAGGCCCGACGGTCCCCAGAGAGC (GTGGTCGCGCGGGCTGTTCCACAGCTCCGGGCCGGGTCAGGGTGGCGGCT) |
| **28** | (AGAGCTCCTTCTGACCCATCCCTTCCCATCTCTTATCCCTGATGTCACTG) ACCGGT ATAACTTCGTATAATGTATGCTATACGAAGTTAT ATGCATGGCC |
| **29** | CGCC ATAACTTCGTATAATGTATGCTATACGAAGTTAT GTCGACCTCGAG CTATATGATCTGCTTCCACTACTGAGCTGAGACCTACAGGAAATCATATC |
| **30** | GTGGGTTGGGCTTATGGAATGACATGAAATCTAGCTTTAGGAGCTTTTTA (GAAGTGTGGAAAACAAGTTTTGGGATATAGGAGTAAAAGGCAGGAAGTTC) |
| **31** | (CCTTAATACACAAAATGATGAAAGAAACTGACTTTCAAATATTACGGGCT) CTCGAGCGGCCGCCAGTGTGATGGATGGTACCTAACTATAACGGTCCT AAGGTAGCGAC CATTTTTTGGGGTTTGTTTCTACCACTCATTCCTCCCT CCCACTTGGTAT |
| **32** | CCCAATTCCCAATTTCTTCCTTCCCTCACTGGGCCCACTAGTAGAATCAT GGTTTGGTGGATTGTGAGCTGAC GGCCGGCC CAACCAGAGCCTCCTGAAGAATGGATCACACAGTTGTTAGACCTCTTCATA TTGAGAATTTGTATCTCCCTCTTCTGTGGGTTTCCCATTGCCCGGCTCCAG |
| | GCATCCCTCAGCTGCACAACTCAC (CCACAATGTCTTCACCTCCACAG) AGGCTGAGCATGGTGGTCAGGGCGAGGACCCCCAGAATCAGAGCTCTGCT |
| **33** | CGGCTGCTGTGGTGGTGCTGATGGTGCTGAGCAGCCCAGGGACTGAGGGC (AGAGACTCTCCCGGTAAGTGCAGGGCCACTGCTCTCCAGAGCCGCCACTC) |
| **34** | (GAGCTCCTTCTGACCCATTCCTTCCCATCTCTTATCCCTGATGTCACTAC) CTCGAG ATAACTTCGTATAATGTATGCTATACGAAGTTAT ATGCATGGCC |
| **35** | TACGAAGTTAT ACGCGTAACTATAACGGTCCTAAGGTAGCGAGCGGCCGC CTATATGATCTGCTTCCACTACTGAGCTGAGACCTACAGGAAATCATATC |
| **36** | AGGGTGAGGGGGCAAGTTAATCTACATAGCCTTTGTGGGTTGGGCTTATG GGCGCGCCATTCGCTACCTTAGGACCGTTATAGTTA (TGAAGTGTGGAAAACAAGTTTTGGGATATAGAGTAAAAGGCAGGAAGTTC) |
| **37** | (TTCAGCTCCAGAGAACATTCCTCACTCATGCACTCACCCACAATGTCTTC) ACCTCCACAGAGGCTGAGCATGGTGGTCAGGGCGAGGACCCCCAGAATCA |
| **38** | GAGTTCCTCCATCACTTCACTGGGTAGCACAGCTGTAACTGTCCAGCCTG (TCCTGGGCTGCAGGTGGTGGGCGTTGCGGGTGGGGCCGGTTAAGGTTCCA) |
| **39** | (TCCCACATCCTATTTTAATTTGCTCCATGTTCTCATCTCCATCAGCACAG) CTCGAG ATAACTTCGTATAATGTATGCTATACGAAGTTAT ATGCATGGCC |
| **40** | ATACGAAGTTAT GCTAGTAACTATAACGGTCCTAAGGTAGCGAGTGGCTT ACAGGTAGGTGCGTGAAGCTTCTACAAGCACAGTTGCCCCCTGGGAAGCA |

The large targeting vectors are used to electroporate MAID5111 ES cells to create modified ES cells comprising a replacement of the endogenous mouse I-A and I-E loci with genomic fragments comprising a chimeric HLA-DQ2.5/H-2A, HLA-DQ8/H-2A, or HLA-DR2/H-2E. Positive ES cells containing deleted endogenous I-A and I-E loci replaced by genomic fragments comprising chimeric loci are identified by a quantitative PCR assay using TAQMAN^{™} probes (Lie and Petropoulos, *supra*).

Positive ES cell clones are then used to implant female mice using the VELOCIMOUSE^{®} method (*supra*) to generate a litter of pups containing a replacement of the endogenous I-A and I-E loci with a chimeric human/mouse locus. Targeted ES cells are used as donor ES cells and introduced into an 8-cell stage mouse embryo by the VELOCIMOUSE^{®} method. Mice bearing a chimeric human/mouse locus are identified by genotyping using a modification of allele assay (Valenzuela et al., *supra*) that detects the presence of a chimeric human/mouse locus.

Mice bearing a chimeric human/mouse locus are bred to a Cre deletor mouse strain (see, e.g., International Patent Application Publication No. WO 2009/114400) in order to remove any loxed neomycin or hygromycin cassette introduced by the targeting vector that is not removed, e.g., at the ES cell stage or in the embryo.

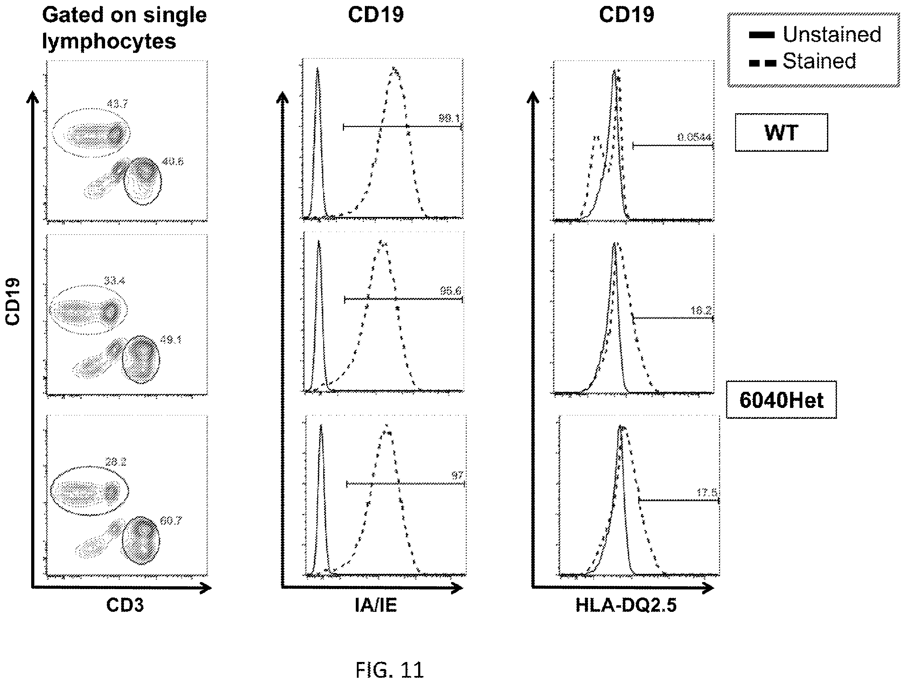

Expression of chimeric human/mouse protein is tested. Specifically, for HLA-DQ2.5/H-2A genetically modified animals, blood from WT or heterozygous humanized HLA-DQ2.5 mice ("6040 HET"; cassette deleted) were drawn into heparin coated capillary tubes and erythrocytes were lysed with ACK lysis buffer. Cell surface expression of human HLA-DQ2.5 was analyzed by FACS using fluorochrome-conjugated anti-CD3 (17A2), anti-CD19 (1D3), anti-I-A/I-E (M15) and anti-HLA-DQ (Tu169) antibodies. Flow cytometry was performed using BD-Fortessa. Expression of both mouse MHC-II (I-A/I-E) and human HLA-DQ2.5 was clearly detectable on the surface of antigen presenting CD19+ mouse B cells (FIG. 11).

## Claims

1. A mouse comprising, at an endogenous MHC II gene locus, a nucleotide sequence encoding a first chimeric human/mouse MHC II α polypeptide and a nucleotide sequence encoding a first chimeric human/mouse MHC II β polypeptide,
wherein a human portion of the first chimeric human/mouse MHC II α polypeptide is encoded by at least a portion of an α chain gene of HLA-DR2, comprises α1 and α2 domains of an HLA-DR2 α polypeptide, and is operably linked to transmembrane and cytoplasmic domains of an endogenous mouse MHC II α polypeptide, and
wherein a human portion of the first chimeric human/mouse MHC II β polypeptide is encoded by at least a portion of a β chain gene of HLA-DR2, comprises β1 and β2 domains of an HLA-DR2 β polypeptide, and is operably linked to transmembrane and cytoplasmic domains of an endogenous mouse MHC II β polypeptide, and
wherein the first chimeric human/mouse MHC II α and first chimeric human/mouse MHC II β polypeptides form an MHC II complex on a surface of a cell of the mouse,
wherein (a) the endogenous mouse MHC II α polypeptide and the endogenous mouse MHC II β polypeptide are from a mouse H-2E α polypeptide and a mouse H-2E β polypeptide, respectively, or (b) the endogenous mouse MHC II α polypeptide and the endogenous mouse MHC II β polypeptide are from a mouse H-2A α polypeptide and a mouse H-2A β polypeptide, respectively.

2. The mouse of claim 1, wherein the nucleotide sequence encoding the first chimeric human/mouse MHC II α polypeptide is operably linked to endogenous mouse MHC II α promoter and regulatory elements, and the nucleotide sequence encoding the first chimeric human/mouse MHC II β polypeptide is operably linked to endogenous mouse MHC II β promoter and regulatory elements.

3. The mouse of claim 1 or 2 comprising, at an endogenous H-2E locus, the nucleotide sequence encoding the first chimeric human/mouse MHC II α polypeptide and the nucleotide sequence encoding the first chimeric human/mouse MHC II β polypeptide,
wherein the human portion of the first chimeric human/mouse MHC II α polypeptide is operably linked to a transmembrane domain and a cytoplasmic domain of an endogenous H-2E α polypeptide, and
wherein the human portion of the first chimeric human/mouse MHC II β polypeptide is operably linked to a transmembrane domain and a cytoplasmic domain of an endogenous H-2E β polypeptide.

4. The mouse of claim 3 comprising, at an endogenous H-2A locus, a nucleotide sequence encoding a second chimeric human/mouse MHC II α polypeptide and a nucleotide sequence encoding a second chimeric human/mouse MHC II β polypeptide,
wherein a human portion of the second chimeric human/mouse MHC II α polypeptide is encoded by at least a portion of an α chain gene of HLA-DQ8.1, comprises α1 and α2 domains of an HLA-DQ8.1 α polypeptide, and is operably linked to transmembrane and cytoplasmic domains of an endogenous H-2A α polypeptide, and
wherein a human portion of the second chimeric human/mouse MHC II β polypeptide is encoded by at least a portion of a β chain gene of HLA-DQ8.1, comprises β1 and β2 domains of an HLA-DQ8.1 β polypeptide, and is operably linked to transmembrane and cytoplasmic domains of an endogenous H-2A β polypeptide.

5. The mouse of claim 3 or 4, wherein
(a) the mouse does not express a functional extracellular domain of a mouse H-2E α polypeptide from an endogenous H-2E locus and/or does not express a functional extracellular domain of a mouse H-2E β polypeptide from an endogenous H-2E locus; and/or
(b) the endogenous H-2E locus lacks an endogenous nucleotide sequence encoding α1 and α2 domains of a mouse H-2E α polypeptide and/or lacks an endogenous nucleotide sequence encoding β1 and β2 domains of a mouse H-2E β polypeptide.

6. The mouse of claim 4 or 5, wherein
(a) the mouse does not express a functional extracellular domain of a mouse H-2A α polypeptide from an endogenous H-2A locus and/or does not express a functional extracellular domain of a mouse H-2A β polypeptide from an endogenous H-2A locus; and/or
(b) the endogenous H-2A locus lacks an endogenous nucleotide sequence encoding αl and α2 domains of a mouse H-2A α polypeptide and/or lacks an endogenous nucleotide sequence encoding β1 and β2 domains of a mouse H-2A β polypeptide.

7. A nucleic acid encoding a chimeric human/mouse MHC II complex, comprising:
a first nucleotide sequence encoding a chimeric human/mouse MHC II α polypeptide and a second nucleotide sequence encoding a chimeric human/mouse MHC II β polypeptide,
wherein a human portion of the chimeric human/mouse MHC II α polypeptide is encoded by at least a portion of an α chain gene of HLA-DR2, comprises α1 and α2 domains of HLA-DR2, and is operably linked to transmembrane and cytoplasmic domains of a mouse MHC II α polypeptide, and
wherein a human portion of the chimeric human/mouse MHC II β polypeptide is encoded by at least a portion of a β chain gene of HLA-DR2, comprises β1 and β2 domains of HLA-DR2, and is operably linked to transmembrane and cytoplasmic domains of a mouse MHC II β polypeptide,
optionally wherein the mouse MHC II α polypeptide is a mouse H-2E α polypeptide, and wherein the mouse MHC II β polypeptide is a mouse H-2E β polypeptide.

8. A method of making a genetically modified mouse comprising:
modifying an endogenous H-2E locus of a mouse to express a chimeric human/mouse MHC II α polypeptide and a chimeric human/mouse MHC II β polypeptide, comprising:
replacing, at an endogenous H-2E locus, at least a nucleotide sequence encoding α1 and α2 domains of a mouse H-2E α polypeptide with at least a nucleotide sequence encoding α1 and α2 domains of a human HLA-DR2 α polypeptide, whereby the nucleotide sequence encoding the α1 and α2 domains of the human HLA-DR2 α polypeptide is operably linked to a nucleotide sequence encoding transmembrane and cytoplasmic domains of the mouse H-2E α polypeptide in the endogenous H-2E locus, and
replacing, at the endogenous H-2E locus, at least a nucleotide sequence encoding β1 and β2 domains of a mouse H-2E β polypeptide with at least a nucleotide sequence encoding β1 and β2 domains of a human HLA-DR2 β polypeptide, whereby the nucleotide sequence encoding the β1 and β2 domains of the human HLA-DR2 β polypeptide is operably linked to a nucleotide sequence encoding transmembrane and cytoplasmic domains of the mouse H-2E β polypeptide in the endogenous H-2E locus;
wherein the mouse expresses a chimeric human/mouse MHC II complex that comprises a chimeric human/mouse MHC II α polypeptide and a chimeric human/mouse MHC II β polypeptide on a surface of a cell of the mouse.

9. A chimeric human/mouse MHC II α polypeptide comprising α1 and α2 domains of a human MHC II α polypeptide of HLA-DR2 operably linked to transmembrane and cytoplasmic domains of a mouse MHC II α polypeptide,
wherein the mouse MHC II α polypeptide is a mouse H-2E α polypeptide.

10. A chimeric human/mouse MHC II β polypeptide comprising β1 and β2 domains of a human MHC II β polypeptide of HLA-DR2 operably linked to transmembrane and cytoplasmic domains of a mouse MHC II β polypeptide,
wherein the mouse MHC II β polypeptide is a mouse H-2E β polypeptide.

11. A chimeric human/mouse MHC II protein complex comprising
(i) a chimeric human/mouse MHC II α polypeptide comprising α1 and α2 domains of a human MHC II α polypeptide of HLA-DR2 operably linked to transmembrane and cytoplasmic domains of a mouse MHC II α polypeptide, wherein the mouse MHC II α polypeptide is a mouse H-2E α polypeptide; and
(ii) a chimeric human/mouse MHC II β polypeptide comprising β1 and β2 domains of a human MHC II β polypeptide of HLA-DR2 operably linked to transmembrane and cytoplasmic domains of a mouse MHC II β polypeptide, wherein the mouse MHC II β polypeptide is a mouse H-2E β polypeptide.

12. A method for identifying a human CD4+ T-cell epitope comprising exposing a mouse of any one of claims 1-6 to an antigen comprising a CD4+ T-cell epitope,
allowing the mouse to mount an immune response,
isolating from the mouse an MHC class II-restricted CD4+ T-cell that binds the CD4+ T-cell epitope; and
identifying the CD4+ T-cell epitope bound by the MHC class II-restricted CD4+ T-cell.

13. An isolated mouse cell comprising (a) a nucleotide sequence encoding a chimeric human/mouse MHC II α polypeptide, wherein a human portion of the chimeric human/mouse MHC II α polypeptide comprises α1 and α2 domains of a human MHC II α polypeptide, wherein the human MHC II α polypeptide is an HLA-DR2 α polypeptide and/or (b) a nucleotide sequence encoding a chimeric human/mouse MHC II β polypeptide, wherein a human portion of the chimeric human/mouse MHC II β polypeptide comprises β1 and β2 domains of a human MHC II β polypeptide, wherein the human MHC II β polypeptide is an HLA-DR2 β polypeptide,
wherein the isolated mouse cell is an antigen presenting cell or an embryonic stem cell; and wherein a mouse portion of the chimeric human/mouse MHC II α polypeptide comprises transmembrane and cytoplasmic domains of a mouse MHC II α polypeptide and/or a mouse portion of the chimeric human/mouse MHC II β polypeptide comprises transmembrane and cytoplasmic domains of a mouse MHC II β polypeptide, and
wherein (a) the mouse MHC II α polypeptide and the mouse MHC II β polypeptide are from a mouse H-2E α polypeptide and a mouse H-2E β polypeptide, respectively, or (b) the mouse MHC II α polypeptide and the mouse MHC II β polypeptide are from a mouse H-2A α polypeptide and a mouse H-2A β polypeptide, respectively.

14. The isolated mouse cell of claim 13, wherein (a) the human MHC II α polypeptide is an HLA-DR2 α polypeptide and the mouse MHC II α polypeptide is a mouse H-2E α polypeptide and/or (b) the human MHC II β polypeptide is an HLA-DR2 β polypeptide and the mouse MHC II β polypeptide is a mouse H-2E β polypeptide.

## Patentansprüche

1. Maus, umfassend eine Nukleotidsequenz, die für ein erstes chimäres Mensch/Maus-MHC-II-α-Polypeptid kodiert, und eine Nukleotidsequenz, die für ein erstes chimäres Mensch/Maus-MHC-II-β-Polypeptid kodiert, an einem endogenen MHC-II-Genlokus,
wobei ein menschlicher Abschnitt des ersten chimären Mensch/Maus-MHC-II-α-Polypeptids von zumindest einem Abschnitt eines α-Kettengens von HLA-DR2 kodiert wird, α1- und α2-Domänen eines HLA-DR2-α-Polypeptids umfasst und operabel an Transmembran- und Zytoplasma-Domänen eines endogenen Maus-MHC-II-α-Polypeptids gebunden ist und
wobei ein menschlicher Abschnitt des ersten chimären Mensch/Maus-MHC-II-β-Polypeptids von zumindest einem Abschnitt eines β-Kettengens von HLA-DR2 kodiert wird, β1- und β2-Domänen eines HLA-DR2-β-Polypeptids umfasst und operabel an Transmembran- und Zytoplasma-Domänen eines endogenen Maus-MHC-II-β-Polypeptids gebunden ist und
wobei das erste chimäre Mensch/Maus-MHC-II-α- und das erste chimäre Mensch/Maus-MHC-II-β-Polypeptid einen MHC-II-Komplex auf einer Oberfläche einer Zelle der Maus bilden,
wobei (a) das endogene Maus-MHC-II-α-Polypeptid und das endogene Maus-MHC-II-β-Polypeptid von einem Maus-H-2E-α-Polypeptid bzw. einem Maus-H-2E-β-Polypeptid stammen oder (b) das endogene Maus-MHC-II-α-Polypeptid und das endogene Maus-MHC-II-β-Polypeptid von einem Maus-H-2A-α-Polypeptid bzw. einem Maus-H-2A-β-Polypeptid stammen.

2. Maus nach Anspruch 1, wobei die Nukleotidsequenz, die für das erste chimäre Mensch/Maus-MHC-II-α-Polypeptid kodiert, operabel an einen endogenen Maus-MHC-II-α-Promotor und -Regulationselemente gebunden ist und die Nukleotidsequenz, die für das erste chimäre Mensch/Maus-MHC-II-β-Polypeptid kodiert, operabel an einen endogenen Maus-MHC-II-β-Promotor und -Regulationselemente gebunden ist.

3. Maus nach Anspruch 1 oder 2, umfassend die Nukleotidsequenz, die für das erste chimäre Mensch/Maus-MHC-II-α-Polypeptid kodiert, und die Nukleotidsequenz, die für das erste chimäre Mensch/Maus-MHC-II-β-Polypeptid kodiert, an einem endogenen H-2E-Locus,
wobei der menschliche Abschnitt des ersten chimären Mensch/Maus-MHC-II-α-Polypeptids operabel an eine Transmembrandomäne und eine Zytoplasmadomäne eines endogenen H-2E-α-Polypeptids gebunden ist und
wobei der menschliche Abschnitt des ersten chimären Mensch/Maus-MHC-II-β-Polypeptids operabel an eine Transmembrandomäne und eine Zytoplasmadomäne eines endogenen H-2E-β-Polypeptids gebunden ist.

4. Maus nach Anspruch 3, umfassend eine Nukleotidsequenz, die für ein zweites chimäres Mensch/Maus-MHC-II-α-Polypeptid kodiert, und eine Nukleotidsequenz, die für ein zweites chimäres Mensch/Maus-MHC-II-β-Polypeptid kodiert, an einem endogenen H-2A-Locus,
wobei ein menschlicher Abschnitt des zweiten chimären Mensch/Maus-MHC-II-α-Polypeptids von zumindest einem Abschnitt eines α-Kettengens von HLA-DQ8.1 kodiert wird, α1- und α2-Domänen eines HLA-DQ8.1-α-Polypeptids umfasst und operabel an Transmembran- und Zytoplasma-Domänen eines endogenen H-2A-α-Polypeptids gebunden ist und
wobei ein menschlicher Abschnitt des zweiten chimären Mensch/Maus-MHC-II-β-Polypeptids von zumindest einem Abschnitt eines β-Kettengens von HLA-DQ8.1 kodiert wird, β1- und β2-Domänen eines HLA-DQ8.1-β-Polypeptids umfasst und operabel an Transmembran- und Zytoplasma-Domänen eines endogenen H-2A-β-Polypeptids gebunden ist.

5. Maus nach Anspruch 3 oder 4, wobei
(a) die Maus keine funktionelle extrazelluläre Domäne eines Maus-H-2E-α-Polypeptids von einem endogenen H-2E-Locus exprimiert und/oder keine funktionelle extrazelluläre Domäne eines Maus-H-2E-β-Polypeptids von einem endogenen H-2E-Locus exprimiert; und/oder
(b) dem endogenen H-2E-Locus eine endogene Nukleotidsequenz fehlt, die für α1- und α2-Domänen eines Maus-H-2E-α-Polypeptids kodiert, und/oder eine endogene Nukleotidsequenz fehlt, die für β1- und β2-Domänen eines Maus-H-2E-β-Polypeptids kodiert.

6. Maus nach Anspruch 4 oder 5, wobei
(a) die Maus keine funktionelle extrazelluläre Domäne eines Maus-H-2A-α-Polypeptids von einem endogenen H-2A-Locus exprimiert und/oder keine funktionelle extrazelluläre Domäne eines Maus-H-2A-β-Polypeptids von einem endogenen H-2A-Locus exprimiert; und/oder
(b) dem endogenen H-2A-Locus eine endogene Nukleotidsequenz fehlt, die für α1- und α2-Domänen eines Maus-H-2A-α-Polypeptids kodiert, und/oder eine endogene Nukleotidsequenz fehlt, die für β1- und β2-Domänen eines Maus-H-2A-β-Polypeptids kodiert.

7. Nukleinsäure, die für einen chimären Mensch/Maus-MHC-II-Komplex kodiert, umfassend:
eine erste Nukleotidsequenz, die für ein chimäres Mensch/Maus-MHC-II-α-Polypeptid kodiert, und eine zweite Nukleotidsequenz, die für ein chimäres Mensch/Maus-MHC-II-β-Polypeptid kodiert,
wobei ein menschlicher Abschnitt des chimären Mensch/Maus-MHC-II-α-Polypeptids von zumindest einem Abschnitt eines α-Kettengens von HLA-DR2 kodiert wird, α1- und α2-Domänen von HLA-DR2 umfasst und operabel an Transmembran- und Zytoplasma-Domänen eines Maus-MHC-II-α-Polypeptids gebunden ist und
wobei ein menschlicher Abschnitt des chimären Mensch/Maus-MHC-II-β-Polypeptids von zumindest einem Abschnitt eines β-Kettengens von HLA-DR2 kodiert wird, β1- und β2-Domänen von HLA-DR2 umfasst und operabel an Transmembran- und Zytoplasma-Domänen eines Maus-MHC-II-β-Polypeptids gebunden ist,
gegebenenfalls wobei das Maus-MHC-II-α-Polypeptid ein Maus-H-2E-α-Polypeptid ist und wobei das Maus-MHC-II-β-Polypeptid ein Maus-H-2E-β-Polypeptid ist.

8. Verfahren zur Herstellung einer genetisch modifizierten Maus, umfassend:
Modifizieren eines endogenen H-2E-Locus einer Maus, um ein chimäres Mensch/Maus-MHC-II-α-Polypeptid und ein chimäres Mensch/Maus-MHC-II-β-Polypeptid zu exprimieren, umfassend:
Ersetzen von zumindest einer Nukleotidsequenz, die für α1- und α2-Domänen eines Maus-H-2E-α-Polypeptids kodiert, an einem endogenen H-2E-Locus mit zumindest einer Nukleotidsequenz, die für α1- und α2-Domänen eines menschlichen HLA-DR2-α-Polypeptids kodiert, wodurch die Nukleotidsequenz, die für α1- und α2-Domänen des menschlichen HLA-DR2-α-Polypeptids kodiert, operabel an eine Nukleotidsequenz, die für Transmembran- und Zytoplasma-Domänen des Maus-H-2E-α-Polypeptids kodiert, in dem endogenen H-2E-Locus gebunden ist, und
Ersetzen von zumindest einer Nukleotidsequenz, die für β1- und β2-Domänen eines Maus-H-2E-β-Polypeptids kodiert, an einem endogenen H-2E-Locus mit zumindest einer Nukleotidsequenz, die für β1- und β2-Domänen eines menschlichen HLA-DR2-β-Polypeptids kodiert, wodurch die Nukleotidsequenz, die für β1- und β2-Domänen des menschlichen HLA-DR2-β-Polypeptids kodiert, operabel an eine Nukleotidsequenz, die für Transmembran- und Zytoplasma-Domänen des Maus-H-2E-β-Polypeptids kodiert, in dem endogenen H-2E-Locus gebunden ist;
wobei die Maus einen chimären Mensch/Maus-MHC-II-Komplex exprimiert, der ein chimäres Mensch/Maus-MHC-II-α-Polypeptid und ein chimäres Mensch/Maus-MHC-II-β-Polypeptid auf einer Oberfläche einer Zelle der Maus umfasst.

9. Chimäres Mensch/Maus-MHC-II-α-Polypeptid, umfassend α1- und α2-Domänen eines menschlichen MHC-II-α-Polypeptids von HLA-DR2, das operabel an Transmembran- und Zytoplasmadomänen eines Maus-MHC-II-α-Polypeptids gebunden ist,
wobei das Maus-MHC-II-α-Polypeptid ein Maus-H-2E-α-Polypeptid ist.

10. Chimäres Mensch/Maus-MHC-II-β-Polypeptid, umfassend β1- und β2-Domänen eines menschlichen MHC-II-β-Polypeptids von HLA-DR2, das operabel an Transmembran- und Zytoplasmadomänen eines Maus-MHC-II-β-Polypeptids gebunden ist,
wobei das Maus-MHC-II-β-Polypeptid ein Maus-H-2E-β-Polypeptid ist.

11. Chimärer Mensch/Maus-MHC-II-Proteinkomplex, umfassend:
(i) ein chimäres Mensch/Maus-MHC-II-α-Polypeptid, umfassend α1- und α2-Domänen eines menschlichen MHC-II-α-Polypeptids von HLA-DR2, das operabel an Transmembran- und Zytoplasmadomänen eines Maus-MHC-II-α-Polypeptids gebunden ist, wobei das Maus-MHC-II-α-Polypeptid ein Maus-H-2E-α-Polypeptid ist; und
(ii) ein chimäres Mensch/Maus-MHC-II-β-Polypeptid, umfassend β1- und β2-Domänen eines menschlichen MHC-II-β-Polypeptids von HLA-DR2, das operabel an Transmembran- und Zytoplasmadomänen eines Maus-MHC-II-β-Polypeptids gebunden ist, wobei das Maus-MHC-II-β-Polypeptid ein Maus-H-2E-β-Polypeptid ist.

12. Verfahren zum Identifizieren eines menschlichen CD4+-T-Zell-Epitops, umfassend:
Aussetzen einer Maus nach einem der Ansprüche 1 bis 6 gegenüber einem Antigen, umfassend ein CD4+-T-Zell-Epitop,
Erzeugenlassen einer Immunantwort in der Maus,
Isolieren einer MHC-Klasse-II-restringierten CD4+-T-Zelle, die das CD4+-T-Zell-Epitop bindet, aus der Maus; und
Identifizieren des durch die MHC-Klasse-II-restringierte CD4+-T-Zelle gebundenen CD4+-T-Zell-Epitops.

13. Isolierte Mauszelle, umfassend (a) eine Nukleotidsequenz, die für ein chimäres Mensch/Maus-MHC-II-α-Polypeptid kodiert, wobei ein menschlicher Abschnitt des chimären Mensch/Maus-MHC-II-α-Polypeptids α1- und α2-Domänen eines menschlichen MHC-II-α-Polypeptids umfasst, wobei das menschliche MHC-II-α-Polypeptid ein HLA-DR2-α-Polypeptid ist, und/oder (b) eine Nukleotidsequenz, die für ein chimäres Mensch/Maus-MHC-II-β-Polypeptid kodiert, wobei ein menschlicher Abschnitt des chimären Mensch/Maus-MHC-II-β-Polypeptids β1- und β2-Domänen eines menschlichen Mensch/Maus-MHC-II-β-Polypeptids umfasst, wobei das menschliche MHC-II-β-Polypeptid ein HLA-DR2-β-Polypeptid ist,
wobei die isolierte Mauszelle eine antigenpräsentierende Zelle oder eine embryonische Stammzelle ist; und
wobei ein Mausabschnitt des chimären Mensch/Maus-MHC-II-α-Polypeptids Transmembran- und Zytoplasmadomänen eines Maus-MHC-II-α-Polypeptids umfasst und/oder ein Mausabschnitt des chimären Mensch/Maus-MHC-II-β-Polypeptids Transmembran- und Zytoplasmadomänen eines Maus-MHC-II-β-Polypeptids umfasst und
wobei (a) das Maus-MHC-II-α-Polypeptid und das Maus-MHC-II-β-Polypeptid von einem Maus-H-2E-α-Polypeptid bzw. einem Maus-H-2E-β-Polypeptid stammen oder (b) das Maus-MHC-II-α-Polypeptid und das Maus-MHC-II-β-Polypeptid von einem Maus-H-2A-α-Polypeptid bzw. einem Maus-H-2A-β-Polypeptid stammen.

14. Isolierte Mauszelle nach Anspruch 13, wobei (a) das menschliche MHC-II-α-Polypeptid ein HLA-DR2-α-Polypeptid ist und das Maus-MHC-II-α-Polypeptid ein Maus-H-2E-α-Polypeptid ist und/oder (b) das menschliche MHC-II-β-Polypeptid ein HLA-DR2-β-Polypeptid ist und das Maus-MHC-II-β-Polypeptid ein Maus-H-2E-β-Polypeptid ist.

## Revendications

1. Souris comprenant, au niveau d'un locus de gène de CMH II endogène, une séquence nucléotidique codant pour un premier polypeptide de CMH II α humain/de souris chimérique et une séquence nucléotidique codant pour un premier polypeptide de CMH II β humain/de souris chimérique,
dans laquelle une partie humaine du premier polypeptide de CMH II α humain/de souris chimérique est codée par au moins une partie d'un gène à chaîne α de HLA-DR2, comprend des domaines α1 et α2 d'un polypeptide de HLA-DR2 α, et est liée de manière opérationnelle à des domaines transmembranaires et cytoplasmiques d'un polypeptide de CMH II α de souris endogène, et
dans laquelle une partie humaine du premier polypeptide de CMH II β humain/de souris chimérique est codée par au moins une partie d'un gène de chaîne β de HLA-DR2, comprend des domaines β1 et β2 d'un polypeptide de HLA-DR2 β, et est liée de manière opérationnelle à des domaines transmembranaires et cytoplasmiques d'un polypeptide de CMH II β de souris endogène, et
dans laquelle le premier polypeptide de CMH II α humain/de souris chimérique et le premier polypeptide de CMH II β humain/de souris chimérique forment un complexe de CMH II sur une surface d'une cellule de la souris ; et
dans laquelle (a) le polypeptide de CMH II α de souris endogène et le polypeptide de CMH II β de souris endogène proviennent d'un polypeptide H-2E α de souris et d'un polypeptide H-2E β de souris, respectivement, ou (b) le polypeptide de CMH II α de souris endogène et le polypeptide de CMH II β de souris endogène proviennent d'un polypeptide H-2A α de souris et d'un polypeptide H-2A β de souris, respectivement.

2. Souris selon la revendication 2, dans laquelle la séquence nucléotidique codant pour le premier polypeptide de CMH II α humain/de souris chimérique est liée de manière opérationnelle au promoteur de CMH II α de souris endogène et à des éléments de régulation, et la séquence nucléotidique codant pour le premier polypeptide de CMH II β humain/de souris chimérique est liée de manière opérationnelle au promoteur de CMH II β de souris endogène et à des éléments de régulation.

3. Souris selon la revendication 1 ou 2 comprenant, au niveau d'un locus de H-2E endogène, la séquence nucléotidique codant pour le premier polypeptide de CMH II α humain/de souris chimérique et la séquence nucléotidique codant pour le premier polypeptide de CMH II β humain/de souris,
dans laquelle la partie humaine du premier polypeptide de CMH II α humain/de souris chimérique est liée de manière opérationnelle à un domaine transmembranaire et à un domaine cytoplasmique d'un polypeptide H-2E α endogène, et
dans laquelle la partie humaine du premier polypeptide de CMH II β humain/de souris chimérique est liée de manière opérationnelle à un domaine transmembranaire et à un domaine cytoplasmique d'un polypeptide H-2E β endogène.

4. Souris selon la revendication 3 comprenant, au niveau d'un locus de H-2A endogène, une séquence nucléotidique codant pour un second polypeptide de CMH II α humain/de souris chimérique et une séquence nucléotidique codant pour un second polypeptide de CMH II β humain/de souris,
dans laquelle une partie humaine du second polypeptide de CMH II α humain/de souris chimérique est codée par au moins une partie d'un gène à chaîne α de HLA-DQ8.1, comprend des domaines α1 et α2 d'un polypeptide de HLA-DQ8.1 α et est liée de manière opérationnelle à des domaines transmembranaires et cytoplasmiques d'un polypeptide de H-2A α endogène, et
dans laquelle une partie humaine du second polypeptide de CMH II β humain/de souris chimérique est codée par au moins une partie d'un gène de chaîne β de HLA-DQ8.1, comprend des domaines β1 et β2 d'un polypeptide de HLA-DQ8.1 β, et est liée de manière opérationnelle à des domaines transmembranaires et cytoplasmiques d'un polypeptide de H-2A β endogène.

5. Souris selon la revendication 3 ou 4, dans laquelle
(a) la souris n'exprime pas un domaine extracellulaire fonctionnel d'un polypeptide de H-2E α de souris à partir d'un locus de H-2E endogène et/ou n'exprime pas un domaine extracellulaire fonctionnel d'un polypeptide de H-2E β de souris à partir d'un locus de H-2E endogène ; et/ou
(b) le locus de H-2E endogène est dépourvu d'une séquence nucléotidique endogène codant pour des domaines α1 et α2 d'un polypeptide de H-2E α de souris, et/ou est dépourvu d'une séquence nucléotidique endogène codant pour des domaines β1 et β2 d'un polypeptide de H-2E β de souris.

6. Souris selon la revendication 4 ou 5, dans laquelle
(a) la souris n'exprime pas un domaine extracellulaire fonctionnel d'un polypeptide de H-2A α de souris à partir d'un locus de H-2A endogène et/ou n'exprime pas un domaine extracellulaire fonctionnel d'un polypeptide de H-2A β de souris à partir d'un locus de H-2A endogène ; et/ou
(b) le locus de H-2A endogène est dépourvu d'une séquence nucléotidique endogène codant pour des domaines α1 et α2 d'un polypeptide de H-2A α de souris, et/ou est dépourvu d'une séquence nucléotidique endogène codant pour des domaines β1 et β2 d'un polypeptide de H-2A β de souris.

7. Acide nucléique codant pour un complexe de CMH II humain/de souris chimérique, comprenant :
une première séquence nucléotidique codant pour un polypeptide de CMH II α humain/de souris chimérique et une seconde séquence nucléotidique codant pour un polypeptide de CMH II β humain/de souris chimérique,
dans lequel une partie humaine du polypeptide de CMH II α humain/de souris chimérique est codée par au moins une partie d'un gène à chaîne α de HLA-DR2, comprend des domaines α1 et α2 de HLA-DR2 et est liée de manière opérationnelle à des domaines transmembranaires et cytoplasmiques d'un polypeptide de CMH II α de souris, et
dans lequel une partie humaine du polypeptide de CMH II β humain/de souris chimérique est codée par au moins une partie d'un gène de chaîne β de HLA-DR2, comprend des domaines β1 et B2 de HLA-DR2, et est liée de manière opérationnelle à des domaines transmembranaires et cytoplasmiques d'un polypeptide de CMH II β de souris,
facultativement dans lequel le polypeptide de CMH II α de souris est un polypeptide de H-2E α de souris, et dans lequel le polypeptide de CMH II β de souris est un polypeptide de H-2E β de souris.

8. Procédé de production d'une souris génétiquement modifiée, comprenant les étapes consistant à :
modifier un locus de H-2E endogène d'une souris pour exprimer un polypeptide de CMH II α humain/de souris chimérique et un polypeptide de CMH II β humain/de souris chimérique, comprenant les étapes consistant à :
remplacer, au niveau d'un locus de H-2E endogène, au moins une séquence nucléotidique codant pour des domaines α1 et α2 d'un polypeptide de H-2E α de souris par au moins une séquence nucléotidique codant pour des domaines α1 et α2 d'un polypeptide de HLA-DR2 α humain, la séquence nucléotidique codant pour les domaines α1 et α2 du polypeptide de HLA-DR2 α humain étant liée de manière opérationnelle à une séquence nucléotidique codant pour des domaines transmembranaires et cytoplasmiques du polypeptide de H-2E α de souris dans le locus de H-2E endogène, et
remplacer, au niveau du locus de H-2E endogène, au moins une séquence nucléotidique codant pour des domaines β1 et β2 d'un polypeptide de H-2E β de souris par au moins une séquence nucléotidique codant pour des domaines β1 et β2 d'un polypeptide de HLA-DR2 β humain, la séquence nucléotidique codant pour les domaines β1 et β2 du polypeptide de HLA-DR2 β humain étant liée de manière opérationnelle à une séquence nucléotidique codant pour des domaines transmembranaires et cytoplasmiques du polypeptide de H-2E β de souris dans le locus de H-2E endogène ;
et dans lequel la souris exprime un complexe de CMH II humain/de souris chimérique qui comprend un polypeptide de CMH II α humain/de souris chimérique et un polypeptide de CMH II β humain/de souris chimérique sur une surface d'une cellule de la souris.

9. Polypeptide de CMH II α humain/de souris chimérique comprenant des domaines α1 et α2 d'un polypeptide de CMH-II α humain de HLA-DR2 liés de manière opérationnelle aux domaines transmembranaires et cytoplasmiques d'un polypeptide de CMH II α de souris,
dans lequel le polypeptide de CMH II α de souris est un polypeptide de H-2E α de souris.

10. Polypeptide de CMH II β humain/de souris chimérique comprenant des domaines β1 et β2 d'un polypeptide de CMH II β humain de HLA-DR2 liés de manière opérationnelle aux domaines transmembranaires et cytoplasmiques d'un polypeptide de CMH II β de souris, et dans lequel le polypeptide de CMH II β de souris est un polypeptide de H-2E β de souris.

11. Complexe protéique de CMH II humain/de souris chimérique, comprenant
(i) un polypeptide de CMH II α humain/de souris chimérique comprenant des domaines α1 et α2 d'un polypeptide de CMH II α humain de HLA-DR2 liés de manière opérationnelle à des domaines transmembranaires et cytoplasmiques d'un polypeptide de CMH II α de souris, dans lequel le polypeptide de CMH II α de souris est un polypeptide de H-2E α de souris ; et
(ii) un polypeptide de CMH II β humain/de souris chimérique comprenant des domaines β1 et β2 d'un polypeptide de CMH II β humain liés de manière opérationnelle aux domaines transmembranaires et cytoplasmiques d'un polypeptide de CMH II β de souris endogène, dans lequel le polypeptide de CMH II β de souris est un polypeptide de H-2E β de souris.

12. Procédé d'identification d'un épitope de lymphocyte T CD4+ humain, comprenant les étapes consistant à
exposer une souris selon l'une quelconque des revendications 1 à 6 à un antigène comprenant un épitope de lymphocyte T CD4+,
permettre à la souris de monter une réponse immunitaire, et
isoler de la souris un lymphocyte T CD4+ restreint par le CMH de classe II qui se lie à l'épitope de lymphocyte T CD4+; et
identifier l'épitope de lymphocyte T CD4+ lié par le lymphocyte T CD4+ restreint par le CMH de classe II.

13. Cellule de souris isolée comprenant (a) une séquence nucléotidique codant pour un polypeptide de CMH II α humain/de souris chimérique, dans laquelle une partie humaine du polypeptide de CMH II α humain/de souris chimérique comprend des domaines α1 et α2 d'un polypeptide de CMH II α humain, dans laquelle le polypeptide de CMH II α humain est un polypeptide de HLA-DR2 α et/ou (b) une séquence nucléotidique codant pour un polypeptide de CMH II β humain/de souris chimérique, dans laquelle une partie humaine du polypeptide de CMH II β humain/de souris chimérique comprend des domaines β1 et β2 d'un polypeptide de CMH II β humain, dans laquelle le polypeptide de CMH II β humain est un polypeptide de HLA-DR2 β,
dans laquelle la cellule de souris isolée est une cellule présentatrice d'antigène ou une cellule souche embryonnaire, et dans laquelle une partie de souris du polypeptide de CMH II α humain/de souris chimérique comprend des domaines transmembranaires et cytoplasmiques d'un polypeptide de CMH II α de souris et/ou une partie de souris du polypeptide de CMH II β humain/de souris chimérique comprend des domaines transmembranaires et cytoplasmiques d'un polypeptide de CMH II β de souris, et
dans laquelle (a) le polypeptide de CMH II α de souris et le polypeptide de CMH II β de souris proviennent d'un polypeptide de H-2E α de souris et d'un polypeptide de H-2E β de souris, respectivement, ou (b) le polypeptide de CMH II α de souris et le polypeptide de CMH II β de souris proviennent d'un polypeptide de H-2A α de souris et d'un polypeptide de H-2A β de souris, respectivement.

14. Cellule de souris isolée selon la revendication 13, dans laquelle (a) le polypeptide de CMH II α humain est un polypeptide de HLA-DR2 α et le polypeptide de CMH II α de souris est un polypeptide H-2E α de souris et/ou (b) le polypeptide de CMH II β humain est un polypeptide de HLA-DR2 β et le polypeptide de CMH II β de souris est un polypeptide de H-2E β de souris.
